(19)

Europäisches
Patentamt
European
Patent Office
Office européen
des brevets

(11)  **EP 3 305 275 A1**

(12)  **EUROPEAN PATENT APPLICATION**
published in accordance with Art. 153(4) EPC

(43) Date of publication:
**11.04.2018 Bulletin 2018/15**

(21) Application number: **16799559.6**

(22) Date of filing: **20.05.2016**

(51) Int Cl.:
*A61K 8/73* (2006.01)          *A23G 1/00* (2006.01)
*A23G 1/30* (2006.01)          *A23G 3/34* (2006.01)
*A23G 4/00* (2006.01)          *A23L 2/52* (2006.01)
*A23L 33/10* (2016.01)          *A23L 33/16* (2016.01)
*A61K 8/24* (2006.01)          *A61Q 11/00* (2006.01)

(86) International application number:
**PCT/JP2016/002470**

(87) International publication number:
**WO 2016/189846 (01.12.2016 Gazette 2016/48)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **26.05.2015   JP 2015106298**

(71) Applicant: **Kabushiki Kaisha Sangi
Tokyo 104-8440 (JP)**

(72) Inventors:
• **TAKAMATSU, Rie
  Tokyo 104-8440 (JP)**
• **OBUKI, Mariko
  Tokyo 104-8440 (JP)**
• **KASUGA, Ayako
  Tokyo 104-8440 (JP)**
• **TAKIKAWA, Rimiko
  Tokyo 104-8440 (JP)**

(74) Representative: **D Young & Co LLP
120 Holborn
London EC1N 2DY (GB)**

(54)  **COMPOSITION FOR ORAL CAVITY AND FOOD PRODUCT, OR BEVERAGE**

(57)   It is an object to provide an oral composition, a food, or a beverage and a remineralizing agent having excellent action of remineralizing demineralized tooth enamel. The present invention is an oral composition, a food, or a beverage comprising sodium chondroitin sulfate and a calcium phosphate. The present invention is a tooth remineralizing agent comprising sodium chondroitin sulfate and a calcium phosphate as active ingredients.

[Figure 1]

EXAMPLE 18

CONTROL SURFACE ← → TREATED SURFACE

**Description**

**Technical Field**

The present invention relates to an oral composition, a food, or a beverage having a tooth remineralizing effect.

**Background Art**

Caries begins with the adhesion of tooth decay bacteria such as *streptococcus mutans* bacteria to a tooth surface and the formation of plaque, and an organic acid produced by the metabolism of foodstuff by the tooth decay bacteria in the plaque demineralizes tooth enamel to cause a state of initial caries. Saliva has the function of remineralizing this demineralized portion by the function of calcium and phosphorus in the saliva and returning the tooth to the original state. If the demineralized tooth can be sufficiently regenerated by remineralization, the occurrence of caries can be suppressed.

Accordingly, dentifrices in which a fluoride and hydroxyapatite that is one of calcium phosphates and has a crystal structure similar to that of the inorganic component of teeth are blended for accelerating the remineralization of teeth are produced and sold.

However, the remineralization of demineralized portions is not sufficient only with saliva and the use of a dentifrice in which a fluoride or hydroxyapatite is blended, and the development of an oral composition, such as a dentifrice, that can sufficiently achieve remineralization is required.

Accordingly, an oral dentifrice in which hydroxyapatite and tricalcium phosphate having a particle diameter of 0.05 $\mu$m to 1.0 $\mu$m are blended and which can restore and protect minute irregularities on tooth surfaces, prevent tooth decay, strengthen dentin, and enhance a whitening effect (Patent Document 1), an oral composition in which a hydroxyapatite fine powder is blended in a water-soluble cellulose solution, thereby being able to allow the hydroxyapatite fine powder to remain long on tooth surfaces (Patent Document 2), an oral composition in which a sugar alcohol such as xylitol and calcium secondary phosphate are used in combination, thereby being able to significantly accelerate remineralization (Patent Document 3), an oral composition in which low crystalline hydroxyapatite is blended and which can prevent diseases and an uncomfortable feeling in the oral cavity by adsorbing to bacteria in the oral cavity for disinfection (Patent Document 4), an oral composition in which a calcium compound such as hydroxyapatite is blended in royal jelly or an extract thereof and which can whiten teeth, prevent tooth decay by remineralization, and prevent periodontal diseases (Patent Document 5), a dentifrice composition in which a calcium compound such as hydroxyapatite is blended in an ultramarine blue composition and which accelerates a tooth remineralizing effect (Patent Document 6), a remineralization accelerating agent comprising a micellar calcium phosphate-phosphopeptide complex and having a cariostatic function (Patent Document 7), a method that can accelerate remineralization and suppress caries by cleaning teeth using a dentifrice comprising a fluoride ion, and then allowing an oral liquid composition comprising a calcium ion to act (Patent Document 8), a dentifrice composition having a pH of 5 to 8 in which tricalcium phosphate is blended as a calcium salt powder having the ability to convert into hydroxyapatite when coming into contact with water in the oral cavity (Patent Document 9), and the like are proposed.

In addition, chewing gums and the like in which xylitol and a calcium phosphate, a noncrystalline calcium phosphate, or a phosphorylated oligosaccharide calcium are blended for accelerating remineralization are also produced, but remineralization is not always sufficient.

Sodium chondroitin sulfate is a white to pale yellow-white powder obtained from the cartilage of a shark or the like, is one of mucopolysaccharides that have high water absorbency and dissolve in water to form a viscous liquid, is present in all tissues including the connective tissue and cartilage of animals, and serves the function of supplying water and nutrition to cells and maintaining water in the cells. Due to such a function, cellular tissue connecting bones to bones is smooth, and the flexibility is maintained, and therefore sodium chondroitin sulfate is widely utilized as drugs and health foods for the treatment and alleviation of joint pain, lower back pain, frozen shoulder, and the like. Sodium chondroitin sulfate is also used as food additives and used in fish sausages, mayonnaise, dressing, and the like as a water retaining agent or an emulsion stabilizer.

For sodium chondroitin sulfate, containing glutamine or sodium chondroitin sulfate to obtain an oral composition having anti-inflammatory action and effective for the prevention and treatment of periodontal diseases such as gingivitis (Patent Document 10), containing chondroitin sulfuric acid or a salt thereof to obtain an oral humectant having an excellent moisturizing effect and an enhanced bad breath preventing effect (Patent Document 11), containing sodium chondroitin sulfate to obtain a liquid oral composition having a plaque formation suppressing effect (Patent Document 12), using sodium chondroitin sulfate as the binder of a dentifrice composition that delivers a medicinal component to gingival grooves and allows the medicinal component to act efficiently in the gingival grooves (Patent Document 13), and the like are proposed. However, sodium chondroitin sulfate has no tooth remineralizing action.

**Prior Art Documents**

**Patent Documents**

Patent Document 1: Japanese unexamined Patent Application Publication No. 9-202717
Patent Document 2: Japanese unexamined Patent Application Publication No. 10-59814
Patent Document 3: Japanese unexamined Patent Application Publication No. 2000-128752
Patent Document 4: Japanese unexamined Patent Application Publication No. 2001-122748
Patent Document 5: Japanese unexamined Patent Application Publication No. 2005-314266
Patent Document 6: Japanese unexamined Patent Application Publication No. 2014-73989
Patent Document 7: Japanese unexamined Patent Application Publication No. 2006-213668
Patent Document 8: Japanese unexamined Patent Application Publication No. 2007-99632
Patent Document 9: Japanese unexamined Patent Application Publication No. 7-223930
Patent Document 10: Japanese unexamined Patent Application Publication No. 63-253018
Patent Document 11: Japanese unexamined Patent Application Publication No. 2006-117563
Patent Document 12: Japanese unexamined Patent Application Publication No. 2009-46449
Patent Document 13: Japanese unexamined Patent Application Publication No. 2009-196987

**Summary of the Invention**

**Object to be Solved by the Invention**

It is an object of the present invention to provide an oral composition, a food, or a beverage and a remineralizing agent having excellent action of remineralizing demineralized tooth enamel.

**Means to Solve the Object**

The present inventors have studied diligently in order to solve the above object, and as a result found that when sodium chondroitin sulfate conventionally known to have only an anti-inflammatory effect, a moisturizing effect, and the like and having no tooth remineralizing action is used together with a calcium phosphate such as hydroxyapatite, tricalcium phosphate, or calcium monohydrogen phosphate and blended in an oral composition such as a dentifrice, a food, or a beverage, the tooth remineralizing effect improves significantly, leading to the completion of the present invention.

Specifically, the present invention is specified by the items shown below.

(1) An oral composition, a food, or a beverage comprising sodium chondroitin sulfate and calcium phosphate.
(2) The oral composition, the food, or the beverage according to the above (1), wherein the calcium phosphate is at least one calcium phosphate selected from the group consisting of hydroxyapatite, tricalcium phosphate, and calcium monohydrogen phosphate.
(3) The oral composition, the food, or the beverage according to the above (1) or (2), wherein a content of the calcium phosphate is 0.0001 to 40% by mass.
(4) The oral composition, the food, or the beverage according to any one of the above (1) to (3), wherein a content of the sodium chondroitin sulfate is 0.0001 to 15% by mass.
(5) The oral composition according to any one of the above (1) to (4), wherein the composition is a paste dentifrice, a powder dentifrice, a liquid dentifrice, a mouthwash, an oral cavity cleaning agent, or a troche.
(6) The food according to any one of the above (1) to (4), wherein the food is a chewing gum, a candy, a chocolate, a yogurt, or a jelly.
(7) The beverage according to any one of the above (1) to (4), wherein the beverage is a carbonated beverage, a lactic acid bacteria beverage, a fruit juice beverage, or a juice.
(8) A tooth remineralizing agent comprising sodium chondroitin sulfate and calcium phosphate as active ingredients.

**Effect of the Invention**

The present invention can exceedingly enhance the action of remineralizing demineralized tooth enamel by comprising sodium chondroitin sulfate and a calcium phosphate such as hydroxyapatite, tricalcium phosphate, or calcium monohydrogen phosphate as active ingredients, and can provide an oral composition, a food, or a beverage and a remineralizing agent having exceedingly excellent remineralizing action by containing these.

**Brief Description of Drawings**

[Figure 1] Figure 1 is a photograph showing the control surface and treated surface of a crown portion in Example 18 by a contact microradiogram (CMR).
[Figure 2] Figure 2 is a photograph showing the control surface and treated surface of a crown portion in Example 60 by a contact microradiogram (CMR).

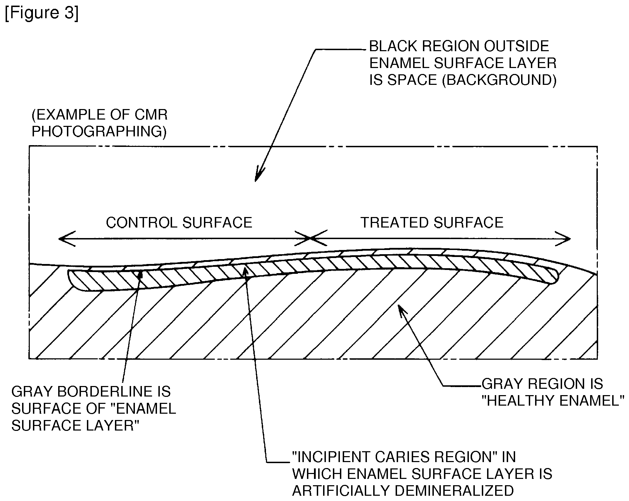

[Figure 3] Figure 3 is a diagram in which the photograph shown in Figure 1 and Figure 2 is drawn using dark India ink, and the explanation of the state of each portion is added.

**Mode of Carrying Out the Invention**

The oral composition, food, or beverage of the present invention is not particularly limited as long as it contains sodium chondroitin sulfate and a calcium phosphate. Examples of the oral composition can include a dentifrice such as a paste dentifrice, a powder dentifrice, and a liquid dentifrice, an oral cleaning agent such as a mouthwash and a gargling tablet, and a troche. Examples of the food can include confectionery such as a chewing gum, a candy, tablet confectionery, a gummy jelly, a chocolate, a biscuit, and a snack, frozen confectionery such as an ice cream, a sherbet, and an ice, bread, a pancake, a dairy product, a meat product such as a ham and a sausage, a fish product such as a boiled fish paste and a tube-shaped fish paste cake, a daily dish, a pudding, a soup, and a jam. Examples of the beverage can include a carbonated beverage, a lactic acid bacteria beverage, a fruit juice beverage, and a juice. The tooth remineralizing agent of the present invention is not particularly limited as long as it contains sodium chondroitin sulfate and a calcium phosphate as active ingredients. Examples of the tooth remineralizing agent of the present invention can include tablet-like, powdery, pasty, liquid, and other remineralizing agents. The calcium phosphate in the present invention is not particularly limited as long as it is a calcium salt of phosphoric acid. Examples of the calcium phosphate in the present invention can include hydroxyapatite, tricalcium phosphate, and calcium monohydrogen phosphate. These calcium phosphates may each be used alone, two or more of these calcium phosphates may be used in combination, and these calcium phosphates may be hydrates or calcium phosphates in which some of phosphorus and calcium are substituted by other elements such as magnesium, zinc, titanium, sodium, and potassium. As the calcium phosphate in the present invention, at least one calcium phosphate selected from the group consisting of hydroxyapatite, tricalcium phosphate, and calcium monohydrogen phosphate is preferred from the viewpoint of remineralization acceleration. The content of the calcium phosphate in the present invention is preferably 0.0001 to 40% by mass, more preferably 0.01 to 30% by mass, of the entire oral composition, food, or beverage from the viewpoint of further improving the remineralizing effect and improving the feeling of use. The content of the calcium phosphate in the remineralizing agent of the present invention when the remineralizing agent is used in the oral cavity as it is preferably 0.0001 to 40% by mass, more preferably 0.01 to 30% by mass, of the entire remineralizing agent from the viewpoint of further improving the remineralizing effect and improving the feeling of use. When the remineralizing agent is added to an oral composition, a food, a beverage, or the like and used, the content of the calcium phosphate in the remineralizing agent of the present invention is preferably set so that the content of the calcium phosphate in the oral composition, the food, the beverage, or the like after the remineralizing agent is added is in the above range. The oral composition, food, or beverage and remineralizing agent of the present invention can significantly improve the remineralization of teeth because sodium chondroitin sulfate is blended together with a calcium phosphate such as hydroxyapatite, tricalcium phosphate, or calcium monohydrogen phosphate.

The hydroxyapatite that is one of the calcium phosphate in the present invention may be hydroxyapatite obtained as natural hard tissue from a fish bone of a food fish such as a salmon, a pig bone, a cow bone, or the like, in addition to hydroxyapatite synthesized by a usual method. Usually, hydroxyapatite is stoichiometrically represented by a composition consisting of $Ca_{10}(PO_4)_6(OH)_2$, but even nonstoichiometric hydroxyapatite not having a Ca/P molar ratio of 1.67 can have an apatite structure as well as exhibiting the properties of hydroxyapatite. For example, synthetic hydroxyapatite having a Ca/P molar ratio of about 1.4 to 1.8 is also included in the hydroxyapatite in the present invention.

The hydroxyapatite used in the present invention may be any of crystalline, low crystalline, and noncrystalline hydroxyapatite but is preferably low crystalline or noncrystalline hydroxyapatite (low crystalline hydroxyapatite and noncrystalline hydroxyapatite are hereinafter referred to as "amorphous hydroxyapatite") in terms of a caries preventing effect. "Low crystalline" refers to crystalline hydroxyapatite in which the X-ray diffraction peak is broader than that of a highly crystalline powder, and "noncrystalline" refers to hydroxyapatite in which the X-ray diffraction pattern shows a broad halo, and a diffraction pattern characteristic of a crystal is not obtained. Such amorphous hydroxyapatite can be obtained, for example, by freeze-drying apatite synthesized by a wet synthesis method or drying the apatite at a temperature of 100°C or less or firing the apatite at a temperature of about 300°C or less.

The content of hydroxyapatite in the present invention is preferably 0.0001 to 40% by mass, more preferably 0.01 to 30% by mass, of the entire oral composition, food, or beverage from the viewpoint of further improving the remineralizing effect and improving the feeling of use. The content of hydroxyapatite in the remineralizing agent of the present invention when the remineralizing agent is used in the oral cavity as it is preferably 0.0001 to 40% by mass, more preferably 0.01 to 30% by mass, of the entire remineralizing agent from the viewpoint of further improving the remineralizing effect and improving the feeling of use. When the remineralizing agent is added to an oral composition, a food, a beverage, or the like and used, the content of hydroxyapatite in the remineralizing agent of the present invention is preferably set so that the content of hydroxyapatite in the oral composition, the food, the beverage, or the like after the remineralizing agent is added is in the above range.

The tricalcium phosphate that is one of the calcium phosphate in the present invention is also referred to as tribasic calcium phosphate, is a compound represented by the chemical formula $Ca_3(PO_4)_2$, and is widely and generally used in foods, sundry goods, the petrochemical industry, and the like including drugs and cosmetics. The tricalcium phosphate used in the present invention is not particularly limited as long as it can be used as a component of an oral composition, a food, or a beverage. Examples of the tricalcium phosphate used in the present invention can include a product conforming to a standard such as Japanese Pharmaceutical Excipients, the Japanese Standards of Quasi-Drug Ingredients, or Japanese Cosmetic Ingredients Codex.

The calcium monohydrogen phosphate that is one of the calcium phosphate in the present invention is also referred to as dibasic calcium phosphate, is a compound represented by the chemical formula $CaHPO_4$ or $CaHPO_4 \cdot 2H_2O$, the dihydrate thereof, and is widely and generally used in drugs, foods, cosmetics, industrial raw materials, and the like. The calcium monohydrogen phosphate used in the present invention is not particularly limited as long as it can be used as a component of an oral composition, a food, or a beverage. Examples of the calcium monohydrogen phosphate used in the present invention can include a product conforming to a standard such as the Japanese Standards of Food Additives, the Japanese Pharmacopoeia, or the Japanese Standards of Quasi-Drug Ingredients.

The content of tricalcium phosphate and calcium monohydrogen phosphate in the present invention is preferably 0.0001 to 40% by mass, more preferably 0.01 to 30% by mass, of the entire oral composition, food, or beverage from the viewpoint of further improving the remineralizing effect and improving the feeling of use. When two or more calcium phosphates are used in combination, the total content of the calcium phosphates is preferably 0.0001 to 40% by mass, more preferably 0.01 to 30% by mass, of the entire oral composition, food, or beverage. When the content of the calcium phosphate is more than 40% by mass in the oral composition, food, or beverage of the present invention, the produced composition is hard, and the production may be difficult, and the quality of the produced composition may be problematic. Particularly regarding a composition comprising water such as a paste dentifrice, a yogurt, or a jelly, the calcium phosphate is cakey due to water, and therefore these points are problems. In addition, even if the content of the calcium phosphate is 40% by mass or more, the extent of improvement in the remineralizing effect is small, and particularly for dibasic calcium phosphate, no improvement in the remineralizing effect is noted.

The sodium chondroitin sulfate used in the present invention is a sodium salt of chondroitin sulfuric acid obtained by extraction from the cartilage of a fish such as a shark or the cartilage of a mammal such as a pig and purification. The sodium chondroitin sulfate used in the present invention is not particularly limited as long as it can be used as a component of an oral composition, a food, or a beverage. White to pale yellow-white sodium chondroitin sulfate can be used, and specifically products from Maruha Nichiro Corporation, SEIKAGAKU CORPORATION, and the like can be illustrated. Examples of the sodium chondroitin sulfate used in the present invention can include a product conforming to a standard such as the Japanese Standards of Food Additives, the Japanese Pharmacopoeia, the Japanese Standards of Quasi-Drug Ingredients, or the Japanese Standards of Cosmetic Ingredients.

The amount of sodium chondroitin sulfate blended in the present invention is preferably 0.0001 to 15.0% by weight, more preferably 0.001 to 5.0% by weight, of the entire oral composition, food, or beverage from the viewpoint of further accelerating remineralization and improving the feeling of use.

The oral composition, food, or beverage and remineralizing agent of the present invention can contain, in addition to the above-described components, additives usually used in these, active ingredients such as various medicinal components, and the like. Examples of the additives can include an abrasive, a humectant, a foaming agent, a thickening agent, an emulsifier, a binding agent, a pH adjusting agent, an organic acid, a colorant, a surfactant, an oil and a fat, an alcohol, a sweetener, an acidulant, a perfume, a seasoning, a vitamin, a mineral, and a preservative. Specific examples of these components are shown below. In addition to these components shown below, an appropriate component according to the purpose, the type of composition, and the like can be further blended.

Examples of the abrasive can include calcium carbonate, calcium pyrophosphate, silica such as abrasive precipitated silica and abrasive gel silica, calcium silicate, aluminum silicate, aluminum oxide, aluminum hydroxide, alumina, zeolite, titanium oxide, zirconium silicate, insoluble sodium metaphosphate, trimagnesium phosphate , magnesium carbonate, calcium sulfate, magnesium sulfate, polymethyl methacrylate, bentonite, and a synthetic resin.

Examples of the humectant can include a polyhydric alcohol such as glycerin, propylene glycol, polyethylene glycol, sorbitol, xylitol, ethylene glycol, 1,3-butylene glycol, and isopropylene glycol.

Examples of the foaming agent can include sodium lauryl sulfate, sodium N-lauroyl sarcosinate, and a nonionic surfactant.

Examples of the thickening agent can include a polysaccharide such as ghatti gum, pullulan, gum arabic, soybean polysaccharide, tamarind seed gum, pectin, carrageenan, a processed eucheuma alga, agar, furcellaran, alginic acid and a derivative thereof (alginic acid and an alginate), guar gum, tara gum, locust bean gum, psyllium seed gum, xanthan gum, *Artemisia sphaerocephala* seed gum, glucomannan, a quince seed, starch, modified starch, processed starch, dextrin, deacylated gellan gum, native gellan gum, curdlan, rhamsan gum, welan gum, macrophomopsis gum, tragacanth gum, karaya gum, microcrystalline cellulose, microfibrous cellulose, fermented cellulose, a carboxymethyl cellulose salt, methyl cellulose, hydroxypropyl cellulose, hydroxymethyl cellulose, chitin, chitosan, casein, dextrin, and gelatin, and starch derived from a plant such as potato, waxy potato, corn, tapioca, rice, wheat, kudzu, and bracken.

The emulsifier is not particularly limited as long as it is used for food. Examples of the emulsifier can include a sucrose fatty acid ester, a glycerin fatty acid ester (for example, a polyglycerin fatty acid ester and a monoglycerin fatty acid ester), a propylene glycol fatty acid ester, a sorbitan fatty acid ester, phospholipid (for example, lecithin, lysolecithin, and lipoprotein), sodium stearoyl lactate, and enzymatically decomposed phospholipid (for example, enzymatically decomposed lecithin).

Examples of the binding agent can include methyl cellulose, propylene glycol alginate ester, pullulan, tragacanth gum, xanthan gum, pectin, furcellaran, chitosan, polyethylene oxide, polyvinylpyrrolidone, polyacrylic acid, polymethacrylic acid, peptone, casein, collagen, albumin, gum arabic, karaya gum, EUDRAGIT, ethyl cellulose, cellulose acetate, sodium polyacrylate, polyvinyl alcohol, polyvinyl acetal-dimethylaminoacetate, and cellulose acetate-dibutyl hydroxypropyl ether.

As the pH adjusting agent, organic acids and alkalis naturally derived or obtained by a microbial fermentation method or chemical synthesis that are usually used in foods can be widely used, and the above-described acidulant and the like are also included. Examples of the pH adjusting agent can include itaconic acid, $\alpha$-ketoglutaric acid, phytic acid, mevalonic acid, adipic acid, citric acid, gluconic acid, succinic acid, glacial acetic acid, tartaric acid, lactic acid, hydrochloric acid, acetic acid, fumaric acid, malic acid, phosphoric acid, acidic pyrophosphoric acid, sodium salts, potassium salts, calcium salts, and ammonium salts of these acids, calcium carbonate, calcium hydroxide, calcium phosphate, monosodium phosphate, disodium phosphate, trisodium phosphate, sodium pyrophosphate, sodium polyphosphate, sodium tripolyphosphate, potassium pyrophosphate, sodium hexametaphosphate, sodium metaphosphate, potassium metaphosphate, monosodium fumarate, sodium hydrogen carbonate, sodium carbonate, potassium hydrogen carbonate, potassium carbonate, ammonium hydrogen carbonate, potassium hydroxide, and sodium hydroxide.

Examples of the organic acid can include an organic acid such as citric acid, isocitric acid, malic acid, acetic acid, oxalic acid, malonic acid, succinic acid, glutaric acid, maleic acid, fumaric acid, aconitic acid, lactic acid, tartaric acid, pyruvic acid, ascorbic acid, aldonic acid, and uronic acid, and ume vinegar, cider vinegar, and a chip, a powder, and an extract of a citrus such as lemon, orange, *Citrus junos,* and *Citrus natsudaidai* containing these.

Examples of the colorant can include a coffee powder, cacao pigment, lac pigment, red cabbage pigment, red daikon pigment, butterfly pea pigment, perilla pigment, hibiscus pigment, grape fruit juice pigment, grape fruit skin pigment, purple sweet potato pigment, purple corn pigment, purple yam pigment, elderberry pigment, cranberry pigment, cherry pigment, hibiscus pigment, blackberry pigment, plum pigment, blueberry pigment, raspberry pigment, boysenberry pigment, tomato pigment, lac pigment, strawberry pigment, cacao pigment, caramel pigment, gardenia pigment, angkhak pigment, cochineal pigment, red beet pigment, grape fruit juice pigment, safflower pigment, annatto pigment, gardenia yellow pigment, turmeric pigment, gardenia yellow, amaranth (Food Red No. 2), erythrosine (Food Red No. 3), allura red AC (Food Red No. 40), new coccine (Food Red No. 102), phloxine (Food Red No. 104), rose bengal (Food Red No. 105), acid red (Food Red No. 106), brilliant blue (Food Blue No. 1), indigo carmine (Food Blue No. 2), spirulina pigment, and gardenia pigment.

Examples of the surfactant can include a sorbitan fatty acid ester, a glycerin fatty acid ester, a decaglycerin fatty acid ester, a polyglycerin fatty acid ester, a propylene glycol-pentaerythritol fatty acid ester, a polyoxyethylene sorbitan fatty acid ester, a polyoxyethylene glycerin fatty acid ester, a polyoxyethylene sorbitol fatty acid ester, a polyethylene glycol fatty acid ester, a polyoxyethylene alkyl ether, polyoxyethylene polyoxypropylene glycol, a polyoxypropylene alkyl ether, a polyoxyethylene polyoxypropylene alkyl ether, a polyoxyethylene alkyl phenyl ether, polyoxyethylene castor oil-hydrogenated castor oil, a polyoxyethylene lanolin-lanolin alcohol-beeswax derivative, a polyoxyethylene alkylamine-fatty acid amide, a polyoxyethylene alkyl phenyl formaldehyde condensate, and a homogeneous polyoxyethylene alkyl ether, which are nonionic surfactants, sodium lauryl sulfate, sodium myristyl sulfate, an alkyl sulfate, a polyoxyethylene alkyl sulfate, a N-acylamino acid and a salt thereof, a N-acylmethyltaurine and a salt thereof, a polyoxyethylene alkyl ether acetate, an alkyl sulfocarboxylate, an $\alpha$-olefin sulfonate, an alkyl phosphate, and a polyoxyethylene alkyl ether phosphate, which are anionic surfactants, an alkylammonium and an alkylbenzylammonium salt, which are cationic surfactants, and acetic acid betaine, imidazolinium betaine, and lecithin, which are amphoteric surfactants. Examples of the nonionic surfactants can include a sucrose fatty acid ester and decaglyceryl laurate.

Examples of the oil and fat components can include liquid paraffin, paraffin, a higher alcohol such as cetyl alcohol and stearyl alcohol, a fatty acid ester such as isopropyl myristate, lanolin, a fatty acid, an ester compound such as octyldodecyl myristate, diisopropyl adipate, hexadecyl isostearate, and decyl oleate, squalane, squalene, a medium chain fatty acid triglyceride, and a silicone.

Examples of the alcohol can include a lower alcohol such as ethanol, propyl alcohol, isopropyl alcohol, butanol, and isobutanol, and a polyhydric alcohol such as ethylene glycol, diethylene glycol, propylene glycol, dipropylene glycol, 1,3-butylene glycol, glycerin, 1,5-pentadiol, sorbitol, and polyethylene glycol.

Examples of the sweetener can include sucrose (sugar), fructose, maltitol, glucose, various oligosaccharides, starch syrup, reduced maltose starch syrup, maltotriose, honey, palatinose, trehalose, lactose, xylose, aspartame, sucrose, L-phenylalanine, stevia, saccharin, acesulfame potassium, licorice, stevioside, and rebaudioside, and further a sugar alcohol such as sorbitol, mannitol, xylitol, erythritol, lactitol, and maltitol, and an oligosaccharide such as a fructooligosaccharide, a galactooligosaccharide, lactosucrose, and a xylooligosaccharide.

Examples of the acidulant can include a food organic acid such as citric acid, lactic acid, malic acid, tartaric acid, succinic acid, and gluconic acid, and a salt such as a sodium salt, a calcium salt, and a potassium salt.

Examples of the flavoring can include one flavoring or a mixture of two or more flavorings selected from a natural flavoring material such as an essential oil, an extract, an oleoresin, a recovered flavor, and an isolated flavoring, and a synthetic flavoring material such as an alcohol, an ester, an aldehyde, a ketone, and a lactone. Examples of the form can include a flavoring agent such as an aqueous flavoring, an oily flavoring, an emulsified flavoring, and a powder flavoring. Specific examples can include a natural flavoring such as lemon oil, orange oil, anise oil, clove oil, capsicum oil, cinnamon oil, grapefruit oil, lime oil, tangerine oil, mandarin oil, bergamot oil, peppermint oil, and spearmint oil, an alcohol such as linalool, geraniol, citronellol, myrcenol, farnesol, hexanol, benzyl alcohol, phenylethyl alcohol, anise alcohol, cinnamic alcohol, anethole, linalool, and eugenol, an ester such as ethyl acetate, butyl acetate, citronellyl acetate, benzyl acetate, linalyl acetate, ethyl propionate, isoamyl propionate, geranyl propionate, isoamyl butyrate, and ethyl isovalerate, an aldehyde and a ketone such as octyl aldehyde, undecyl aldehyde, $\alpha$-hexylcinnamaldehyde, nonadienal, octanal, citral, perillaldehyde, phenyl aldehyde, cinnamic aldehyde, vanillin, L-carvone, acetophenone, ionone, damascenone, maltol, benzylacetone, methyl heptyl ketone, and methyl decyl ketone, a lactone such as $\delta$-decalactone, $\gamma$-undecalactone, and sclareolide, a hydrocarbon such as limonene, pinene, and caryophyllene, and an acid such as acetic acid, propionic acid, 2-methylbutyric acid, and cinnamic acid.

Examples of the seasoning can include common salt, sodium glutamate, inosinic acid, and guanylic acid.

Examples of the vitamin can include vitamin C, vitamin D, vitamin E, vitamin A, and vitamin B12.

Examples of the mineral can include calcium, magnesium, iron, zinc, and copper.

Examples of the preservative can include sorbic acid and a salt thereof, benzoic acid and a salt thereof, dehydroacetic acid and a salt thereof, a p-hydroxybenzoate, propionic acid and a salt thereof, sodium acetate, sodium sulfite, sodium hyposulfite, sulfur dioxide, ethanol, glycine, polylysine, protamine, lysozyme, chitosan, a pectin decomposition product, an extract of a plant such as yucca, mustard, wasabi, hop, and moso bamboo, hinokitiol, natamycin, nisin, and a chlorine-based disinfectant such as sodium hypochlorite and a high test bleaching powder, and an oxygen-based disinfectant such as hydrogen peroxide.

Examples of other medicinal components can include allantoin, tocopherol acetate, isopropyl phenol, triclosan, chlorhexidine, chlorophyll, flavonoid, tranexamic acid, hinokitiol, cetylpyridinium chloride, sodium fluoride, stannous fluoride, sodium monofluorophosphate, dextranase, mutanase, protease, aminocaproic acid, glycyrrhizic acid, glycyrrhetic acid, azulene, allantoin, lysozyme chloride, a phellodendron bark extract, polyphosphoric acid, sodium chloride, an aloe squeezed juice, *Gynostemma pentaphyllum,* ginseng, an active oxygen removing agent, an antioxidant, an anti-inflammatory analgesic, an antihistamine, an antipruritic, a disinfectant, a vitamin preparation, and a hormone preparation.

For the amounts of these optional components blended, these optional components are appropriately used in ranges that do not hinder the effect of the present invention and are pharmaceutically allowable. In the production of the oral composition, food, or beverage of the present invention, sodium chondroitin sulfate, a calcium phosphate such as hydroxyapatite, tricalcium phosphate, or calcium monohydrogen phosphate, and other components may be added in any process in the production process.

**Examples**

Paste dentifrices (Examples 1 to 78), liquid dentifrices (Examples 79 to 96), mouthwashes (Examples 97 to 108), chewing gums (Examples 109 to 117), troches (Examples 118 to 132), candies (Examples 133 to 144), and beverages (Examples 145 to 153) in which sodium chondroitin sulfate and hydroxyapatite, tricalcium phosphate, or calcium monohydrogen phosphate were blended were prepared with blends in Table 1 to Table 15, Table 23 to Table 25, Table 30 to Table 32, Table 35 to Table 37, Table 39 to Table 41, Table 43 to Table 45, and Table 47 to Table 49 and subjected to a remineralization test.

[Sodium Chondroitin Sulfate]

For the sodium chondroitin sulfate, JSQI sodium chondroitin sulfate (Maruha Nichiro Foods, Inc.) was used.

[Hydroxyapatite]

A phosphoric acid aqueous solution having a concentration of 30% by mass was dropped into a calcium hydroxide suspension under stirring until a pH of 10 was reached, and the produced gel-like substance was allowed to stand at room temperature for 1 day for aging. Then, the gel-like substance was filtered by a glass filter, and the remaining substance was dried in air at 100°C to obtain a hydroxyapatite powder. The obtained hydroxyapatite powder had a maximum particle diameter of about 40 $\mu$m, a minimum particle diameter of about 0.05 $\mu$m, and an average particle diameter of about 5 $\mu$m. This hydroxyapatite powder was used.

[Tricalcium Phosphate (Tribasic Calcium Phosphate]

For the tricalcium phosphate, food additive: tricalcium phosphate (Taihei Chemical Industrial Co. Ltd.) was used.

[Calcium Monohydrogen Phosphate (Dibasic Calcium Phosphate)]

For the calcium monohydrogen phosphate, food additive: calcium monohydrogen phosphate (YONEYAMA CHEMICAL INDUSTRY CO., LTD.) was used.
As Comparative Examples, paste dentifrices, liquid dentifrices, mouthwashes, chewing gums, troches, candies, and beverages in which sodium chondroitin sulfate and hydroxyapatite, tricalcium phosphate, or calcium monohydrogen phosphate were each blended were prepared with blends in Table 16 to Table 22, Table 26 to Table 29, Table 33, Table 34, Table 38, Table 42, Table 46, Table 50, and Table 51 and subjected to a remineralization test.

1. Paste Dentifrices

[Table 1]

|  | Example 1 | Example 2 | Example 3 | Example 4 | Example 5 |
|---|---|---|---|---|---|
| Sodium chondroitin sulfate | 0.0001 | 0.05 | 15.0 | 0.01 | 0.1 |
| Hydroxyapatite | 40.0 | 40.0 | 40.0 | 30.0 | 30.0 |
| Glycerin | 20.0 | 20.0 | 20.0 | 20.0 | 20.0 |
| Carboxymethyl cellulose | 2.0 | 2.0 | 2.0 | 2.0 | 2.0 |
| Silica | 5.0 | 5.0 | 5.0 | 5.0 | 5.0 |
| Polyethylene glycol | 3.0 | 3.0 | 3.0 | 3.0 | 3.0 |
| Sodium lauryl sulfate | 1.0 | 1.0 | 1.0 | 1.0 | 1.0 |
| Cetylpyridinium chloride | 0.1 | 0.1 | 0.1 | 0.1 | 0.1 |
| Menthol | 0.5 | 0.5 | 0.5 | 0.5 | 0.5 |
| Purified water | Balance | Balance | Balance | Balance | Balance |
| Total | 100.0 | 100.0 | 100.0 | 100.0 | 100.0 |

[Table 2]

|  | Example 6 | Example 7 | Example 8 | Example 9 | Example 10 |
|---|---|---|---|---|---|
| Sodium chondroitin sulfate | 0.05 | 5.0 | 0.001 | 0.05 | 0.5 |
| Hydroxyapatite | 20.0 | 20.0 | 10.0 | 10.0 | 10.0 |
| Glycerin | 20.0 | 20.0 | 20.0 | 10.0 | 10.0 |

(continued)

|  | Example 6 | Example 7 | Example 8 | Example 9 | Example 10 |
|---|---|---|---|---|---|
| Carboxymethyl cellulose | 2.0 | 2.0 | 2.0 | 2.0 | 2.0 |
| Silica | 5.0 | 5.0 | 5.0 | 5.0 | 5.0 |
| Polyethylene glycol | 3.0 | 3.0 | 3.0 | 3.0 | 3.0 |
| Sodium lauryl sulfate | 1.0 | 1.0 | 1.0 | 1.0 | 1.0 |
| Cetylpyridinium chloride | 0.1 | 0.1 | 0.1 | 0.1 | 0.1 |
| Menthol | 0.5 | 0.5 | 0.5 | 0.5 | 0.5 |
| Purified water | Balance | Balance | Balance | Balance | Balance |
| Total | 100.0 | 100.0 | 100.0 | 100.0 | 100.0 |

[Table 3]

|  | Example 11 | Example 12 | Example 13 | Example 14 | Example 15 | Example 16 |
|---|---|---|---|---|---|---|
| Sodium chondroitin sulfate | 0.0001 | 0.01 | 0.1 | 1.0 | 10.0 | 15.0 |
| Hydroxyapatite | 5.0 | 5.0 | 5.0 | 5.0 | 5.0 | 5.0 |
| Glycerin | 20.0 | 20.0 | 20.0 | 20.0 | 10.0 | 10.0 |
| Carboxymethyl cellulose | 2.0 | 2.0 | 2.0 | 2.0 | 2.0 | 2.0 |
| Silica | 5.0 | 5.0 | 5.0 | 5.0 | 5.0 | 5.0 |
| Polyethylene glycol | 3.0 | 3.0 | 3.0 | 3.0 | 3.0 | 3.0 |
| Sodium lauryl sulfate | 1.0 | 1.0 | 1.0 | 1.0 | 1.0 | 1.0 |
| Cetylpyridinium chloride | 0.1 | 0.1 | 0.1 | 0.1 | 0.1 | 0.1 |
| Menthol | 0.5 | 0.5 | 0.5 | 0.5 | 0.5 | 0.5 |
| Purified water | Balance | Balance | Balance | Balance | Balance | Balance |
| Total | 100.0 | 100.0 | 100.0 | 100.0 | 100.0 | 100.0 |

[Table 4]

|  | Example 17 | Example 18 | Example 19 | Example 20 | Example 21 |
|---|---|---|---|---|---|
| Sodium chondroitin sulfate | 0.05 | 0.5 | 5.0 | 0.01 | 1.0 |
| Hydroxyapatite | 1.0 | 1.0 | 1.0 | 0.1 | 0.1 |
| Glycerin | 20.0 | 20.0 | 20.0 | 20.0 | 10.0 |
| Carboxymethyl cellulose | 2.0 | 2.0 | 2.0 | 2.0 | 2.0 |
| Silica | 5.0 | 5.0 | 5.0 | 5.0 | 5.0 |
| Polyethylene glycol | 3.0 | 3.0 | 3.0 | 3.0 | 3.0 |
| Sodium lauryl sulfate | 1.0 | 1.0 | 1.0 | 1.0 | 1.0 |
| Cetylpyridinium chloride | 0.1 | 0.1 | 0.1 | 0.1 | 0.1 |
| Menthol | 0.5 | 0.5 | 0.5 | 0.5 | 0.5 |
| Purified water | Balance | Balance | Balance | Balance | Balance |
| Total | 100.0 | 100.0 | 100.0 | 100.0 | 100.0 |

[Table 5]

| | Example 22 | Example 23 | Example 24 | Example 25 | Example 26 |
|---|---|---|---|---|---|
| Sodium chondroitin sulfate | 0.001 | 0.1 | 0.0001 | 0.05 | 15.0 |
| Hydroxyapatite | 0.01 | 0.01 | 0.0001 | 0.0001 | 0.0001 |
| Glycerin | 10.0 | 20.0 | 20.0 | 20.0 | 20.0 |
| Carboxymethyl cellulose | 2.0 | 2.0 | 2.0 | 2.0 | 2.0 |
| Silica | 5.0 | 5.0 | 5.0 | 5.0 | 5.0 |
| Polyethylene glycol | 3.0 | 3.0 | 3.0 | 3.0 | 3.0 |
| Sodium lauryl sulfate | 1.0 | 1.0 | 1.0 | 1.0 | 1.0 |
| Cetylpyridinium chloride | 0.1 | 0.1 | 0.1 | 0.1 | 0.1 |
| Menthol | 0.5 | 0.5 | 0.5 | 0.5 | 0.5 |
| Purified water | Balance | Balance | Balance | Balance | Balance |
| Total | 100.0 | 100.0 | 100.0 | 100.0 | 100.0 |

[Table 6]

| | Example 27 | Example 28 | Example 29 | Example 30 | Example 31 |
|---|---|---|---|---|---|
| Sodium chondroitin sulfate | 0.0001 | 0.05 | 15.0 | 0.01 | 0.1 |
| Tricalcium phosphate | 40.0 | 40.0 | 40.0 | 30.0 | 30.0 |
| Glycerin | 20.0 | 20.0 | 20.0 | 20.0 | 20.0 |
| Carboxymethyl cellulose | 2.0 | 2.0 | 2.0 | 2.0 | 2.0 |
| Silica | 5.0 | 5.0 | 5.0 | 5.0 | 5.0 |
| Polyethylene glycol | 3.0 | 3.0 | 3.0 | 3.0 | 3.0 |
| Sodium lauryl sulfate | 1.0 | 1.0 | 1.0 | 1.0 | 1.0 |
| Cetylpyridinium chloride | 0.1 | 0.1 | 0.1 | 0.1 | 0.1 |
| Menthol | 0.5 | 0.5 | 0.5 | 0.5 | 0.5 |
| Purified water | Balance | Balance | Balance | Balance | Balance |
| Total | 100.0 | 100.0 | 100.0 | 100.0 | 100.0 |

[Table 7]

| | Example 32 | Example 33 | Example 34 | Example 35 | Example 36 |
|---|---|---|---|---|---|
| Sodium chondroitin sulfate | 0.05 | 5.0 | 0.001 | 0.05 | 0.5 |
| Tricalcium phosphate | 20.0 | 20.0 | 10.0 | 10.0 | 10.0 |
| Glycerin | 20.0 | 20.0 | 20.0 | 10.0 | 10.0 |
| Carboxymethyl cellulose | 2.0 | 2.0 | 2.0 | 2.0 | 2.0 |
| Silica | 5.0 | 5.0 | 5.0 | 5.0 | 5.0 |
| Polyethylene glycol | 3.0 | 3.0 | 3.0 | 3.0 | 3.0 |
| Sodium lauryl sulfate | 1.0 | 1.0 | 1.0 | 1.0 | 1.0 |
| Cetylpyridinium chloride | 0.1 | 0.1 | 0.1 | 0.1 | 0.1 |
| Menthol | 0.5 | 0.5 | 0.5 | 0.5 | 0.5 |

(continued)

|  | Example 32 | Example 33 | Example 34 | Example 35 | Example 36 |
|---|---|---|---|---|---|
| Purified water | Balance | Balance | Balance | Balance | Balance |
| Total | 100.0 | 100.0 | 100.0 | 100.0 | 100.0 |

[Table 8]

|  | Example 37 | Example 38 | Example 39 | Example 40 | Example 41 | Example 42 |
|---|---|---|---|---|---|---|
| Sodium chondroitin sulfate | 0.0001 | 0.01 | 0.1 | 1.0 | 10.0 | 15.0 |
| Tricalcium phosphate | 5.0 | 5.0 | 5.0 | 5.0 | 5.0 | 5.0 |
| Glycerin | 20.0 | 20.0 | 20.0 | 20.0 | 10.0 | 10.0 |
| Carboxymethyl cellulose | 2.0 | 2.0 | 2.0 | 2.0 | 2.0 | 2.0 |
| Silica | 5.0 | 5.0 | 5.0 | 5.0 | 5.0 | 5.0 |
| Polyethylene glycol | 3.0 | 3.0 | 3.0 | 3.0 | 3.0 | 3.0 |
| Sodium lauryl sulfate | 1.0 | 1.0 | 1.0 | 1.0 | 1.0 | 1.0 |
| Cetylpyridinium chloride | 0.1 | 0.1 | 0.1 | 0.1 | 0.1 | 0.1 |
| Menthol | 0.5 | 0.5 | 0.5 | 0.5 | 0.5 | 0.5 |
| Purified water | Balance | Balance | Balance | Balance | Balance | Balance |
| Total | 100.0 | 100.0 | 100.0 | 100.0 | 100.0 | 100.0 |

[Table 9]

|  | Example 43 | Example 44 | Example 45 | Example 46 | Example 47 |
|---|---|---|---|---|---|
| Sodium chondroitin sulfate | 0.05 | 0.5 | 5.0 | 0.01 | 1.0 |
| Tricalcium phosphate | 1.0 | 1.0 | 1.0 | 0.1 | 0.1 |
| Glycerin | 20.0 | 20.0 | 20.0 | 20.0 | 10.0 |
| Carboxymethyl cellulose | 2.0 | 2.0 | 2.0 | 2.0 | 2.0 |
| Silica | 5.0 | 5.0 | 5.0 | 5.0 | 5.0 |
| Polyethylene glycol | 3.0 | 3.0 | 3.0 | 3.0 | 3.0 |
| Sodium lauryl sulfate | 1.0 | 1.0 | 1.0 | 1.0 | 1.0 |
| Cetylpyridinium chloride | 0.1 | 0.1 | 0.1 | 0.1 | 0.1 |
| Menthol | 0.5 | 0.5 | 0.5 | 0.5 | 0.5 |
| Purified water | Balance | Balance | Balance | Balance | Balance |
| Total | 100.0 | 100.0 | 100.0 | 100.0 | 100.0 |

[Table 10]

|  | Example 48 | Example 49 | Example 50 | Example 51 | Example 52 |
|---|---|---|---|---|---|
| Sodium chondroitin sulfate | 0.001 | 0.1 | 0.0001 | 0.05 | 15.0 |
| Tricalcium phosphate | 0.01 | 0.01 | 0.0001 | 0.0001 | 0.0001 |
| Glycerin | 10.0 | 20.0 | 20.0 | 20.0 | 20.0 |

(continued)

|  | Example 48 | Example 49 | Example 50 | Example 51 | Example 52 |
|---|---|---|---|---|---|
| Carboxymethyl cellulose | 2.0 | 2.0 | 2.0 | 2.0 | 2.0 |
| Silica | 5.0 | 5.0 | 5.0 | 5.0 | 5.0 |
| Polyethylene glycol | 3.0 | 3.0 | 3.0 | 3.0 | 3.0 |
| Sodium lauryl sulfate | 1.0 | 1.0 | 1.0 | 1.0 | 1.0 |
| Cetylpyridinium chloride | 0.1 | 0.1 | 0.1 | 0.1 | 0.1 |
| Menthol | 0.5 | 0.5 | 0.5 | 0.5 | 0.5 |
| Purified water | Balance | Balance | Balance | Balance | Balance |
| Total | 100.0 | 100.0 | 100.0 | 100.0 | 100.0 |

[Table 11]

|  | Example 53 | Example 54 | Example 55 | Example 56 | Example 57 |
|---|---|---|---|---|---|
| Sodium chondroitin sulfate | 0.0001 | 0.05 | 15.0 | 0.01 | 0.1 |
| Calcium monohydrogen phosphate | 40.0 | 40.0 | 40.0 | 30.0 | 30.0 |
| Glycerin | 20.0 | 20.0 | 20.0 | 20.0 | 20.0 |
| Carboxymethyl cellulose | 2.0 | 2.0 | 2.0 | 2.0 | 2.0 |
| Silica | 5.0 | 5.0 | 5.0 | 5.0 | 5.0 |
| Polyethylene glycol | 3.0 | 3.0 | 3.0 | 3.0 | 3.0 |
| Sodium lauryl sulfate | 1.0 | 1.0 | 1.0 | 1.0 | 1.0 |
| Cetylpyridinium chloride | 0.1 | 0.1 | 0.1 | 0.1 | 0.1 |
| Menthol | 0.5 | 0.5 | 0.5 | 0.5 | 0.5 |
| Purified water | Balance | Balance | Balance | Balance | Balance |
| Total | 100.0 | 100.0 | 100.0 | 100.0 | 100.0 |

[Table 12]

|  | Example 58 | Example 59 | Example 60 | Example 61 | Example 62 |
|---|---|---|---|---|---|
| Sodium chondroitin sulfate | 0.05 | 5.0 | 0.001 | 0.05 | 0.5 |
| Calcium monohydrogen phosphate | 20.0 | 20.0 | 10.0 | 10.0 | 10.0 |
| Glycerin | 20.0 | 20.0 | 20.0 | 10.0 | 10.0 |
| Carboxymethyl cellulose | 2.0 | 2.0 | 2.0 | 2.0 | 2.0 |
| Silica | 5.0 | 5.0 | 5.0 | 5.0 | 5.0 |
| Polyethylene glycol | 3.0 | 3.0 | 3.0 | 3.0 | 3.0 |
| Sodium lauryl sulfate | 1.0 | 1.0 | 1.0 | 1.0 | 1.0 |
| Cetylpyridinium chloride | 0.1 | 0.1 | 0.1 | 0.1 | 0.1 |
| Menthol | 0.5 | 0.5 | 0.5 | 0.5 | 0.5 |
| Purified water | Balance | Balance | Balance | Balance | Balance |
| Total | 100.0 | 100.0 | 100.0 | 100.0 | 100.0 |

[Table 13]

| | Example 63 | Example 64 | Example 65 | Example 66 | Example 67 | Example 68 |
|---|---|---|---|---|---|---|
| Sodium chondroitin sulfate | 0.0001 | 0.01 | 0.1 | 1.0 | 10.0 | 15.0 |
| Calcium monohydrogen phosphate | 5.0 | 5.0 | 5.0 | 5.0 | 5.0 | 5.0 |
| Glycerin | 20.0 | 20.0 | 20.0 | 20.0 | 10.0 | 10.0 |
| Carboxymethyl cellulose | 2.0 | 2.0 | 2.0 | 2.0 | 2.0 | 2.0 |
| Silica | 5.0 | 5.0 | 5.0 | 5.0 | 5.0 | 5.0 |
| Polyethylene glycol | 3.0 | 3.0 | 3.0 | 3.0 | 3.0 | 3.0 |
| Sodium lauryl sulfate | 1.0 | 1.0 | 1.0 | 1.0 | 1.0 | 1.0 |
| Cetylpyridinium chloride | 0.1 | 0.1 | 0.1 | 0.1 | 0.1 | 0.1 |
| Menthol | 0.5 | 0.5 | 0.5 | 0.5 | 0.5 | 0.5 |
| Purified water | Balance | Balance | Balance | Balance | Balance | Balance |
| Total | 100.0 | 100.0 | 100.0 | 100.0 | 100.0 | 100.0 |

[Table 14]

| | Example 69 | Example 70 | Example 71 | Example 72 | Example 73 |
|---|---|---|---|---|---|
| Sodium chondroitin sulfate | 0.05 | 0.5 | 5.0 | 0.01 | 1.0 |
| Calcium monohydrogen phosphate | 1.0 | 1.0 | 1.0 | 0.1 | 0.1 |
| Glycerin | 20.0 | 20.0 | 20.0 | 20.0 | 10.0 |
| Carboxymethyl cellulose | 2.0 | 2.0 | 2.0 | 2.0 | 2.0 |
| Silica | 5.0 | 5.0 | 5.0 | 5.0 | 5.0 |
| Polyethylene glycol | 3.0 | 3.0 | 3.0 | 3.0 | 3.0 |
| Sodium lauryl sulfate | 1.0 | 1.0 | 1.0 | 1.0 | 1.0 |
| Cetylpyridinium chloride | 0.1 | 0.1 | 0.1 | 0.1 | 0.1 |
| Menthol | 0.5 | 0.5 | 0.5 | 0.5 | 0.5 |
| Purified water | Balance | Balance | Balance | Balance | Balance |
| Total | 100.0 | 100.0 | 100.0 | 100.0 | 100.0 |

[Table 15]

| | Example 74 | Example 75 | Example 76 | Example 77 | Example 78 |
|---|---|---|---|---|---|
| Sodium chondroitin sulfate | 0.001 | 0.1 | 0.0001 | 0.05 | 15.0 |
| Calcium monohydrogen phosphate | 0.01 | 0.01 | 0.0001 | 0.0001 | 0.0001 |
| Glycerin | 10.0 | 20.0 | 20.0 | 20.0 | 20.0 |
| Carboxymethyl cellulose | 2.0 | 2.0 | 2.0 | 2.0 | 2.0 |
| Silica | 5.0 | 5.0 | 5.0 | 5.0 | 5.0 |
| Polyethylene glycol | 3.0 | 3.0 | 3.0 | 3.0 | 3.0 |

(continued)

|  | Example 74 | Example 75 | Example 76 | Example 77 | Example 78 |
|---|---|---|---|---|---|
| Sodium lauryl sulfate | 1.0 | 1.0 | 1.0 | 1.0 | 1.0 |
| Cetylpyridinium chloride | 0.1 | 0.1 | 0.1 | 0.1 | 0.1 |
| Menthol | 0.5 | 0.5 | 0.5 | 0.5 | 0.5 |
| Purified water | Balance | Balance | Balance | Balance | Balance |
| Total | 100.0 | 100.0 | 100.0 | 100.0 | 100.0 |

[Table 16]

| | Comparative Example 1 | Comparative Example 2 | Comparative Example 3 | Comparative Example 4 | Comparative Example 5 | Comparative Example 6 |
|---|---|---|---|---|---|---|
| Sodium chondroitin sulfate | 0.0001 | 0.01 | 0.1 | 1.0 | 10.0 | 15.0 |
| Glycerin | 20.0 | 20.0 | 20.0 | 20.0 | 20.0 | 20.0 |
| Carboxymethyl cellulose | 2.0 | 2.0 | 2.0 | 2.0 | 2.0 | 2.0 |
| Silica | 5.0 | 5.0 | 5.0 | 5.0 | 5.0 | 5.0 |
| Polyethylene glycol | 3.0 | 3.0 | 3.0 | 3.0 | 3.0 | 3.0 |
| Sodium lauryl sulfate | 1.0 | 1.0 | 1.0 | 1.0 | 1.0 | 1.0 |
| Cetylpyridinium chloride | 0.1 | 0.1 | 0.1 | 0.1 | 0.1 | 0.1 |
| Menthol | 0.5 | 0.5 | 0.5 | 0.5 | 0.5 | 0.5 |
| Purified water | Balance | Balance | Balance | Balance | Balance | Balance |
| Total | 100.0 | 100.0 | 100.0 | 100.0 | 100.0 | 100.0 |

[Table 17]

|  | Comparative Example 7 | Comparative Example 8 | Comparative Example 9 | Comparative Example 10 | Comparative Example 11 |
|---|---|---|---|---|---|
| Hydroxyapatite | 0.0001 | 0.01 | 0.1 | 1.0 | 5.0 |
| Glycerin | 20.0 | 20.0 | 20.0 | 20.0 | 20.0 |
| Carboxymethyl cellulose | 2.0 | 2.0 | 2.0 | 2.0 | 2.0 |
| Silica | 5.0 | 5.0 | 5.0 | 5.0 | 5.0 |
| Polyethylene glycol | 3.0 | 3.0 | 3.0 | 3.0 | 3.0 |
| Sodium lauryl sulfate | 1.0 | 1.0 | 1.0 | 1.0 | 1.0 |
| Cetylpyridinium chloride | 0.1 | 0.1 | 0.1 | 0.1 | 0.1 |
| Menthol | 0.5 | 0.5 | 0.5 | 0.5 | 0.5 |
| Purified water | Balance | Balance | Balance | Balance | Balance |
| Total | 100.0 | 100.0 | 100.0 | 100.0 | 100.0 |

[Table 18]

|  | Comparative Example 12 | Comparative Example 13 | Comparative Example 14 | Comparative Example 15 |
|---|---|---|---|---|
| Hydroxyapatite | 10.0 | 20.0 | 30.0 | 40.0 |
| Glycerin | 20.0 | 20.0 | 20.0 | 20.0 |
| Carboxymethyl cellulose | 2.0 | 2.0 | 2.0 | 2.0 |
| Silica | 5.0 | 5.0 | 5.0 | 5.0 |
| Polyethylene glycol | 3.0 | 3.0 | 3.0 | 3.0 |
| Sodium lauryl sulfate | 1.0 | 1.0 | 1.0 | 1.0 |
| Cetylpyridinium chloride | 0.1 | 0.1 | 0.1 | 0.1 |
| Menthol | 0.5 | 0.5 | 0.5 | 0.5 |
| Purified water | Balance | Balance | Balance | Balance |
| Total | 100.0 | 100.0 | 100.0 | 100.0 |

[Table 19]

|  | Comparative Example 16 | Comparative Example 17 | Comparative Example 18 | Comparative Example 19 | Comparative Example 20 |
|---|---|---|---|---|---|
| Tricalcium phosphate | 0.0001 | 0.01 | 0.1 | 1.0 | 5.0 |
| Glycerin | 20.0 | 20.0 | 20.0 | 20.0 | 20.0 |

(continued)

|  | Comparative Example 16 | Comparative Example 17 | Comparative Example 18 | Comparative Example 19 | Comparative Example 20 |
|---|---|---|---|---|---|
| Carboxymethyl cellulose | 2.0 | 2.0 | 2.0 | 2.0 | 2.0 |
| Silica | 5.0 | 5.0 | 5.0 | 5.0 | 5.0 |
| Polyethylene glycol | 3.0 | 3.0 | 3.0 | 3.0 | 3.0 |
| Sodium lauryl sulfate | 1.0 | 1.0 | 1.0 | 1.0 | 1.0 |
| Cetylpyridinium chloride | 0.1 | 0.1 | 0.1 | 0.1 | 0.1 |
| Menthol | 0.5 | 0.5 | 0.5 | 0.5 | 0.5 |
| Purified water | Balance | Balance | Balance | Balance | Balance |
| Total | 100.0 | 100.0 | 100.0 | 100.0 | 100.0 |

[Table 20]

|  | Comparative Example 21 | Comparative Example 22 | Comparative Example 23 | Comparative Example 24 |
|---|---|---|---|---|
| Tricalcium phosphate | 10.0 | 20.0 | 30.0 | 40.0 |
| Glycerin | 20.0 | 20.0 | 20.0 | 20.0 |
| Carboxymethyl cellulose | 2.0 | 2.0 | 2.0 | 2.0 |
| Silica | 5.0 | 5.0 | 5.0 | 5.0 |
| Polyethylene glycol | 3.0 | 3.0 | 3.0 | 3.0 |
| Sodium lauryl sulfate | 1.0 | 1.0 | 1.0 | 1.0 |
| Cetylpyridinium chloride | 0.1 | 0.1 | 0.1 | 0.1 |
| Menthol | 0.5 | 0.5 | 0.5 | 0.5 |
| Purified water | Balance | Balance | Balance | Balance |
| Total | 100.0 | 100.0 | 100.0 | 100.0 |

[Table 21]

|  | Comparative Example 25 | Comparative Example 26 | Comparative Example 27 | Comparative Example 28 | Comparative Example 29 |
|---|---|---|---|---|---|
| Calcium monohydrogen phosphate | 0.0001 | 0.01 | 0.1 | 1.0 | 5.0 |
| Glycerin | 20.0 | 20.0 | 20.0 | 20.0 | 20.0 |
| Carboxymethyl cellulose | 2.0 | 2.0 | 2.0 | 2.0 | 2.0 |

(continued)

| | Comparative Example 25 | Comparative Example 26 | Comparative Example 27 | Comparative Example 28 | Comparative Example 29 |
|---|---|---|---|---|---|
| Silica | 5.0 | 5.0 | 5.0 | 5.0 | 5.0 |
| Polyethylene glycol | 3.0 | 3.0 | 3.0 | 3.0 | 3.0 |
| Sodium lauryl sulfate | 1.0 | 1.0 | 1.0 | 1.0 | 1.0 |
| Cetylpyridinium chloride | 0.1 | 0.1 | 0.1 | 0.1 | 0.1 |
| Menthol | 0.5 | 0.5 | 0.5 | 0.5 | 0.5 |
| Purified water | Balance | Balance | Balance | Balance | Balance |
| Total | 100.0 | 100.0 | 100.0 | 100.0 | 100.0 |

[Table 22]

| | Comparative Example 30 | Comparative Example 31 | Comparative Example 32 | Comparative Example 33 |
|---|---|---|---|---|
| Calcium monohydrogen phosphate | 10.0 | 20.0 | 30.0 | 40.0 |
| Glycerin | 20.0 | 20.0 | 20.0 | 20.0 |
| Carboxymethyl cellulose | 2.0 | 2.0 | 2.0 | 2.0 |
| Silica | 5.0 | 5.0 | 5.0 | 5.0 |
| Polyethylene glycol | 3.0 | 3.0 | 3.0 | 3.0 |
| Sodium lauryl sulfate | 1.0 | 1.0 | 1.0 | 1.0 |
| Cetylpyridinium chloride | 0.1 | 0.1 | 0.1 | 0.1 |
| Menthol | 0.5 | 0.5 | 0.5 | 0.5 |
| Purified water | Balance | Balance | Balance | Balance |
| Total | 100.0 | 100.0 | 100.0 | 100.0 |

2. Liquid Dentifrices

[Table 23]

| | Example 79 | Example 80 | Example 81 | Example 82 | Example 83 | Example 84 |
|---|---|---|---|---|---|---|
| Sodium chondroitin sulfate | 0.0001 | 10.0 | 0.01 | 5.0 | 0.1 | 1.0 |
| Hydroxyapatite | 10.0 | 5.0 | 1.0 | 0.1 | 0.01 | 0.0001 |
| Xylitol | 10.0 | 10.0 | 10.0 | 10.0 | 10.0 | 10.0 |
| Carrageenan | 1.0 | 1.0 | 1.0 | 1.0 | 1.0 | 1.0 |
| Glycerin | 10.0 | 10.0 | 10.0 | 10.0 | 10.0 | 10.0 |
| Menthol | 0.4 | 0.4 | 0.4 | 0.4 | 0.4 | 0.4 |
| Purified water | Balance | Balance | Balance | Balance | Balance | Balance |

(continued)

| | Example 79 | Example 80 | Example 81 | Example 82 | Example 83 | Example 84 |
|---|---|---|---|---|---|---|
| Total | 100.0 | 100.0 | 100.0 | 100.0 | 100.0 | 100.0 |

[Table 24]

| | Example 85 | Example 86 | Example 87 | Example 88 | Example 89 | Example 90 |
|---|---|---|---|---|---|---|
| Sodium chondroitin sulfate | 0.0001 | 10.0 | 0.01 | 5.0 | 0.1 | 1.0 |
| Tricalcium phosphate | 10.0 | 5.0 | 1.0 | 0.1 | 0.01 | 0.0001 |
| Xylitol | 10.0 | 10.0 | 10.0 | 10.0 | 10.0 | 10.0 |
| Carrageenan | 1.0 | 1.0 | 1.0 | 1.0 | 1.0 | 1.0 |
| Glycerin | 10.0 | 10.0 | 10.0 | 10.0 | 10.0 | 10.0 |
| Menthol | 0.4 | 0.4 | 0.4 | 0.4 | 0.4 | 0.4 |
| Purified water | Balance | Balance | Balance | Balance | Balance | Balance |
| Total | 100.0 | 100.0 | 100.0 | 100.0 | 100.0 | 100.0 |

[Table 25]

| | Example 91 | Example 92 | Example 93 | Example 94 | Example 95 | Example 96 |
|---|---|---|---|---|---|---|
| Sodium chondroitin sulfate | 0.0001 | 10.0 | 0.01 | 5.0 | 0.1 | 1.0 |
| Calcium monohydrogen phosphate | 10.0 | 5.0 | 1.0 | 0.1 | 0.01 | 0.0001 |
| Xylitol | 10.0 | 10.0 | 10.0 | 10.0 | 10.0 | 10.0 |
| Carrageenan | 1.0 | 1.0 | 1.0 | 1.0 | 1.0 | 1.0 |
| Glycerin | 10.0 | 10.0 | 10.0 | 10.0 | 10.0 | 10.0 |
| Menthol | 0.4 | 0.4 | 0.4 | 0.4 | 0.4 | 0.4 |
| Purified water | Balance | Balance | Balance | Balance | Balance | Balance |
| Total | 100.0 | 100.0 | 100.0 | 100.0 | 100.0 | 100.0 |

[Table 26]

| | Comparative Example 34 | Comparative Example 35 | Comparative Example 36 |
|---|---|---|---|
| Sodium chondroitin sulfate | 0.0001 | 1.0 | 10.0 |
| Xylitol | 10.0 | 10.0 | 10.0 |
| Carrageenan | 1.0 | 1.0 | 1.0 |
| Glycerin | 10.0 | 10.0 | 10.0 |
| Menthol | 0.4 | 0.4 | 0.4 |
| Purified water | Balance | Balance | Balance |
| Total | 100.0 | 100.0 | 100.0 |

[Table 27]

| | Comparative Example 37 | Comparative Example 38 | Comparative Example 39 | Comparative Example 40 | Comparative Example 41 | Comparative Example 42 |
|---|---|---|---|---|---|---|
| Hydroxyapatite | 10.0 | 5.0 | 1.0 | 0.1 | 0.01 | 0.0001 |
| Xylitol | 10.0 | 10.0 | 10.0 | 10.0 | 10.0 | 10.0 |
| Carrageenan | 1.0 | 1.0 | 1.0 | 1.0 | 1.0 | 1.0 |
| Glycerin | 10.0 | 10.0 | 10.0 | 10.0 | 10.0 | 10.0 |
| Menthol | 0.4 | 0.4 | 0.4 | 0.4 | 0.4 | 0.4 |
| Purified water | Balance | Balance | Balance | Balance | Balance | Balance |
| Total | 100.0 | 100.0 | 100.0 | 100.0 | 100.0 | 100.0 |

[Table 28]

| | Comparative Example 43 | Comparative Example 44 | Comparative Example 45 | Comparative Example 46 | Comparative Example 47 | Comparative Example 48 |
|---|---|---|---|---|---|---|
| Tricalcium phosphate | 10.0 | 5.0 | 1.0 | 0.1 | 0.01 | 0.0001 |
| Xylitol | 10.0 | 10.0 | 10.0 | 10.0 | 10.0 | 10.0 |
| Carrageenan | 1.0 | 1.0 | 1.0 | 1.0 | 1.0 | 1.0 |
| Glycerin | 10.0 | 10.0 | 10.0 | 10.0 | 10.0 | 10.0 |
| Menthol | 0.4 | 0.4 | 0.4 | 0.4 | 0.4 | 0.4 |
| Purified water | Balance | Balance | Balance | Balance | Balance | Balance |
| Total | 100.0 | 100.0 | 100.0 | 100.0 | 100.0 | 100.0 |

[Table 29]

| | Comparative Example 49 | Comparative Example 50 | Comparative Example 51 | Comparative Example 52 | Comparative Example 53 | Comparative Example 54 |
|---|---|---|---|---|---|---|
| Calcium monohydrogen phosphate | 10.0 | 5.0 | 1.0 | 0.1 | 0.01 | 0.0001 |
| Xylitol | 10.0 | 10.0 | 10.0 | 10.0 | 10.0 | 10.0 |
| Carrageenan | 1.0 | 1.0 | 1.0 | 1.0 | 1.0 | 1.0 |
| Glycerin | 10.0 | 10.0 | 10.0 | 10.0 | 10.0 | 10.0 |
| Menthol | 0.4 | 0.4 | 0.4 | 0.4 | 0.4 | 0.4 |
| Purified water | Balance | Balance | Balance | Balance | Balance | Balance |
| Total | 100.0 | 100.0 | 100.0 | 100.0 | 100.0 | 100.0 |

3. Mouthwashes

[Table 30]

|  | Example 97 | Example 98 | Example 99 | Example 100 |
|---|---|---|---|---|
| Sodium chondroitin sulfate | 0.0001 | 0.2 | 0.001 | 2.0 |
| Hydroxyapatite | 0.1 | 0.01 | 0.001 | 0.0001 |
| Ethyl alcohol | 10.0 | 10.0 | 10.0 | 10.0 |
| Sodium lauryl sulfate | 1.0 | 1.0 | 1.0 | 1.0 |
| Glycerin | 10.0 | 10.0 | 10.0 | 10.0 |
| Menthol | 0.4 | 0.4 | 0.4 | 0.4 |
| Purified water | Balance | Balance | Balance | Balance |
| Total | 100.0 | 100.0 | 100.0 | 100.0 |

[Table 31]

|  | Example 101 | Example 102 | Example 103 | Example 104 |
|---|---|---|---|---|
| Sodium chondroitin sulfate | 0.0001 | 0.2 | 0.001 | 2.0 |
| Tricalcium phosphate | 0.1 | 0.01 | 0.001 | 0.0001 |
| Ethyl alcohol | 10.0 | 10.0 | 10.0 | 10.0 |
| Sodium lauryl sulfate | 1.0 | 1.0 | 1.0 | 1.0 |
| Glycerin | 10.0 | 10.0 | 10.0 | 10.0 |
| Menthol | 0.4 | 0.4 | 0.4 | 0.4 |
| Purified water | Balance | Balance | Balance | Balance |
| Total | 100.0 | 100.0 | 100.0 | 100.0 |

[Table 32]

|  | Example 105 | Example 106 | Example 107 | Example 108 |
|---|---|---|---|---|
| Sodium chondroitin sulfate | 0.0001 | 0.2 | 0.001 | 2.0 |
| Calcium monohydrogen phosphate | 0.1 | 0.01 | 0.001 | 0.0001 |
| Ethyl alcohol | 10.0 | 10.0 | 10.0 | 10.0 |
| Sodium lauryl sulfate | 1.0 | 1.0 | 1.0 | 1.0 |
| Glycerin | 10.0 | 10.0 | 10.0 | 10.0 |
| Menthol | 0.4 | 0.4 | 0.4 | 0.4 |
| Purified water | Balance | Balance | Balance | Balance |
| Total | 100.0 | 100.0 | 100.0 | 100.0 |

[Table 33]

|  | Comparative Example 55 | Comparative Example 56 |
|---|---|---|
| Sodium chondroitin sulfate | 0.0001 | 2.0 |

(continued)

|  | Comparative Example 55 | Comparative Example 56 |
|---|---|---|
| Ethyl alcohol | 10.0 | 10.0 |
| Sodium lauryl sulfate | 1.0 | 1.0 |
| Glycerin | 10.0 | 10.0 |
| Menthol | 0.4 | 0.4 |
| Purified water | Balance | Balance |
| Total | 100.0 | 100.0 |

(continued)

|  | Comparative Example 55 | Comparative Example 56 |
|---|---|---|

[Table 34]

| | Comparative Example 57 | Comparative Example 58 | Comparative Example 59 | Comparative Example 60 | Comparative Example 61 | Comparative Example 62 |
|---|---|---|---|---|---|---|
| Hydroxyapatite | 0.0001 | 0.1 | - | - | - | - |
| Tricalcium phosphate | - | - | 0.0001 | 0.1 | - | - |
| Calcium monohydrogen phosphate | - | - | - | - | 0.0001 | 0.1 |
| Ethyl alcohol | 10.0 | 10.0 | 10.0 | 10.0 | 10.0 | 10.0 |
| Sodium lauryl sulfate | 1.0 | 1.0 | 1.0 | 1.0 | 1.0 | 1.0 |
| Glycerin | 10.0 | 10.0 | 10.0 | 10.0 | 10.0 | 10.0 |
| Menthol | 0.4 | 0.4 | 0.4 | 0.4 | 0.4 | 0.4 |
| Purified water | Balance | Balance | Balance | Balance | Balance | Balance |
| Total | 100.0 | 100.0 | 100.0 | 100.0 | 100.0 | 100.0 |

4. Chewing gums

[Table 35]

|  | Example 109 | Example 110 | Example 111 |
|---|---|---|---|
| Sodium chondroitin sulfate | 2.0 | 0.2 | 0.001 |
| Hydroxyapatite | 0.1 | 1.0 | 8.0 |
| Gum base | 28.0 | 28.0 | 28.0 |
| Xylitol | 30.0 | 30.0 | 30.0 |
| Palatinit | 21.0 | 21.0 | 21.0 |
| Maltitol | 3.8 | 3.8 | 3.8 |
| Softening agent | 0.8 | 0.8 | 0.8 |
| Flavoring | 1.0 | 1.0 | 1.0 |
| Reduced maltose starch syrup | Balance | Balance | Balance |
| Total | 100.0 | 100.0 | 100.0 |

[Table 36]

|  | Example 112 | Example 113 | Example 114 |
|---|---|---|---|
| Sodium chondroitin sulfate | 2.0 | 0.2 | 0.001 |
| Tricalcium phosphate | 0.1 | 1.0 | 8.0 |
| Gum base | 28.0 | 28.0 | 28.0 |
| Xylitol | 30.0 | 30.0 | 30.0 |
| Palatinit | 21.0 | 21.0 | 21.0 |
| Maltitol | 3.8 | 3.8 | 3.8 |
| Softening agent | 0.8 | 0.8 | 0.8 |
| Flavoring | 1.0 | 1.0 | 1.0 |
| Reduced maltose starch syrup | Balance | Balance | Balance |
| Total | 100.0 | 100.0 | 100.0 |

[Table 37]

|  | Example 115 | Example 116 | Example 117 |
|---|---|---|---|
| Sodium chondroitin sulfate | 2.0 | 0.2 | 0.001 |
| Calcium monohydrogen phosphate | 0.1 | 1.0 | 8.0 |
| Gum base | 28.0 | 28.0 | 28.0 |
| Xylitol | 30.0 | 30.0 | 30.0 |
| Palatinit | 21.0 | 21.0 | 21.0 |
| Maltitol | 3.8 | 3.8 | 3.8 |
| Softening agent | 0.8 | 0.8 | 0.8 |
| Flavoring | 1.0 | 1.0 | 1.0 |

(continued)

|  | Example 115 | Example 116 | Example 117 |
|---|---|---|---|
| Reduced maltose starch syrup | Balance | Balance | Balance |
| Total | 100.0 | 100.0 | 100.0 |

[Table 38]

| | Comparative Example 63 | Comparative Example 64 | Comparative Example 65 | Comparative Example 66 | Comparative Example 67 | Comparative Example 68 | Comparative Example 69 |
|---|---|---|---|---|---|---|---|
| Sodium chondroitin sulfate | 2.0 | - | - | - | - | - | - |
| Hydroxyapatite | - | 0.1 | 8.0 | - | - | - | - |
| Tricalcium phosphate | - | - | - | 0.1 | 8.0 | - | - |
| Calcium monohydrogen phosphate | - | - | - | - | - | 0.1 | 8.0 |
| Gum base | 28.0 | 28.0 | 28.0 | 28.0 | 28.0 | 28.0 | 28.0 |
| Xylitol | 30.0 | 30.0 | 30.0 | 30.0 | 30.0 | 30.0 | 30.0 |
| Palatinit | 21.0 | 21.0 | 21.0 | 21.0 | 21.0 | 21.0 | 21.0 |
| Maltitol | 3.8 | 3.8 | 3.8 | 3.8 | 3.8 | 3.8 | 3.8 |
| Softening agent | 0.8 | 0.8 | 0.8 | 0.8 | 0.8 | 0.8 | 0.8 |
| Flavoring | 1.0 | 1.0 | 1.0 | 1.0 | 1.0 | 1.0 | 1.0 |
| Reduced maltose starch syrup | Balance | Balance | Balance | Balance | Balance | Balance | Balance |
| Total | 100.0 | 100.0 | 100.0 | 100.0 | 100.0 | 100.0 | 100.0 |

5. Troches

[Table 39]

| | Example 118 | Example 119 | Example 120 | Example 121 | Example 122 |
|---|---|---|---|---|---|
| Sodium chondroitin sulfate | 0.2 | 0.001 | 0.0001 | 0.01 | 1.0 |
| Hydroxyapatite | 30.0 | 15.0 | 1.0 | 0.01 | 0.001 |
| Calcium gluconate | 3.0 | 3.0 | 3.0 | 3.0 | 3.0 |
| Xylitol | 20.0 | 20.0 | 20.0 | 20.0 | 20.0 |
| Palatinit | 30.0 | 30.0 | 30.0 | 30.0 | 30.0 |
| Sorbitol | 5.0 | 5.0 | 5.0 | 5.0 | 5.0 |
| CMCNa | 1.0 | 1.0 | 1.0 | 1.0 | 1.0 |
| Citric acid | 2.5 | 2.5 | 2.5 | 2.5 | 2.5 |
| Flavoring | 1.5 | 1.5 | 1.5 | 1.5 | 1.5 |
| Sucrose fatty acid ester | Balance | Balance | Balance | Balance | Balance |
| Total | 100.0 | 100.0 | 100.0 | 100.0 | 100.0 |

[Table 40]

| | Example 123 | Example 124 | Example 125 | Example 126 | Example 127 |
|---|---|---|---|---|---|
| Sodium chondroitin sulfate | 0.2 | 0.001 | 0.0001 | 0.01 | 1.0 |
| Tricalcium phosphate | 30.0 | 15.0 | 1.0 | 0.01 | 0.001 |
| Calcium gluconate | 3.0 | 3.0 | 3.0 | 3.0 | 3.0 |
| Xylitol | 20.0 | 20.0 | 20.0 | 20.0 | 20.0 |
| Palatinit | 30.0 | 30.0 | 30.0 | 30.0 | 30.0 |
| Sorbitol | 5.0 | 5.0 | 5.0 | 5.0 | 5.0 |
| CMCNa | 1.0 | 1.0 | 1.0 | 1.0 | 1.0 |
| Citric acid | 2.5 | 2.5 | 2.5 | 2.5 | 2.5 |
| Flavoring | 1.5 | 1.5 | 1.5 | 1.5 | 1.5 |
| Sucrose fatty acid ester | Balance | Balance | Balance | Balance | Balance |
| Total | 100.0 | 100.0 | 100.0 | 100.0 | 100.0 |

[Table 41]

| | Example 128 | Example 129 | Example 130 | Example 131 | Example 132 |
|---|---|---|---|---|---|
| Sodium chondroitin sulfate | 0.2 | 0.001 | 0.0001 | 0.01 | 1.0 |
| Calcium monohydrogen phosphate | 30.0 | 15.0 | 1.0 | 0.01 | 0.001 |
| Calcium gluconate | 3.0 | 3.0 | 3.0 | 3.0 | 3.0 |
| Xylitol | 20.0 | 20.0 | 20.0 | 20.0 | 20.0 |
| Palatinit | 30.0 | 30.0 | 30.0 | 30.0 | 30.0 |
| Sorbitol | 5.0 | 5.0 | 5.0 | 5.0 | 5.0 |

(continued)

|  | Example 128 | Example 129 | Example 130 | Example 131 | Example 132 |
|---|---|---|---|---|---|
| CMCNa | 1.0 | 1.0 | 1.0 | 1.0 | 1.0 |
| Citric acid | 2.5 | 2.5 | 2.5 | 2.5 | 2.5 |
| Flavoring | 1.5 | 1.5 | 1.5 | 1.5 | 1.5 |
| Sucrose fatty acid ester | Balance | Balance | Balance | Balance | Balance |
| Total | 100.0 | 100.0 | 100.0 | 100.0 | 100.0 |

[Table 42]

| | Comparative Example 70 | Comparative Example 71 | Comparative Example 72 | Comparative Example 73 | Comparative Example 74 | Comparative Example 75 | Comparative Example 76 |
|---|---|---|---|---|---|---|---|
| Sodium chondroitin sulfate | 1.0 | - | - | - | - | - | - |
| Hydroxyapatite | - | 30.0 | 0.001 | - | - | - | - |
| Tricalcium phosphate | - | - | - | 30.0 | 0.001 | - | - |
| Calcium monohydrogen phosphate | - | - | - | - | - | 30.0 | 0.001 |
| Calcium gluconate | 3.0 | 3.0 | 3.0 | 3.0 | 3.0 | 3.0 | 3.0 |
| Xylitol | 20.0 | 20.0 | 20.0 | 20.0 | 20.0 | 20.0 | 20.0 |
| Palatinit | 30.0 | 30.0 | 30.0 | 30.0 | 30.0 | 30.0 | 30.0 |
| Sorbitol | 5.0 | 5.0 | 5.0 | 5.0 | 5.0 | 5.0 | 5.0 |
| CMCNa | 1.0 | 1.0 | 1.0 | 1.0 | 1.0 | 1.0 | 1.0 |
| Citric acid | 2.5 | 2.5 | 2.5 | 2.5 | 2.5 | 2.5 | 2.5 |
| Flavoring | 1.5 | 1.5 | 1.5 | 1.5 | 1.5 | 1.5 | 1.5 |
| Sucrose fatty acid ester | Balance | Balance | Balance | Balance | Balance | Balance | Balance |
| Total | 100.0 | 100.0 | 100.0 | 100.0 | 100.0 | 100.0 | 100.0 |

6. Candies

[Table 43]

| | Example 133 | Example 134 | Example 135 | Example 136 |
|---|---|---|---|---|
| Sodium chondroitin sulfate | 1.0 | 0.01 | 0.1 | 10.0 |
| Hydroxyapatite | 20.0 | 10.0 | 1.0 | 0.1 |
| Palatinit | 40.0 | 40.0 | 40.0 | 40.0 |
| Maltitol | 20.0 | 20.0 | 20.0 | 20.0 |
| Aspartame | 3.0 | 3.0 | 3.0 | 3.0 |
| Dextrin | 2.0 | 2.0 | 2.0 | 2.0 |
| Citric acid | 0.5 | 0.5 | 0.5 | 0.5 |
| Reduced maltose starch syrup | Balance | Balance | Balance | Balance |
| Total | 100.0 | 100.0 | 100.0 | 100.0 |

[Table 44]

| | Example 137 | Example 138 | Example 139 | Example 140 |
|---|---|---|---|---|
| Sodium chondroitin sulfate | 1.0 | 0.01 | 0.1 | 10.0 |
| Tricalcium phosphate | 20.0 | 10.0 | 1.0 | 0.1 |
| Palatinit | 40.0 | 40.0 | 40.0 | 40.0 |
| Maltitol | 20.0 | 20.0 | 20.0 | 20.0 |
| Aspartame | 3.0 | 3.0 | 3.0 | 3.0 |
| Dextrin | 2.0 | 2.0 | 2.0 | 2.0 |
| Citric acid | 0.5 | 0.5 | 0.5 | 0.5 |
| Reduced maltose starch syrup | Balance | Balance | Balance | Balance |
| Total | 100.0 | 100.0 | 100.0 | 100.0 |

[Table 45]

| | Example 141 | Example 142 | Example 143 | Example 144 |
|---|---|---|---|---|
| Sodium chondroitin sulfate | 1.0 | 0.01 | 0.1 | 10.0 |
| Calcium monohydrogen phosphate | 20.0 | 10.0 | 1.0 | 0.1 |
| Palatinit | 40.0 | 40.0 | 40.0 | 40.0 |
| Maltitol | 20.0 | 20.0 | 20.0 | 20.0 |
| Aspartame | 3.0 | 3.0 | 3.0 | 3.0 |
| Dextrin | 2.0 | 2.0 | 2.0 | 2.0 |
| Citric acid | 0.5 | 0.5 | 0.5 | 0.5 |
| Reduced maltose starch syrup | Balance | Balance | Balance | Balance |
| Total | 100.0 | 100.0 | 100.0 | 100.0 |

[Table 46]

| | Comparative Example 77 | Comparative Example 78 | Comparative Example 79 | Comparative Example 80 | Comparative Example 81 | Comparative Example 82 | Comparative Example 83 |
|---|---|---|---|---|---|---|---|
| Sodium chondroitin sulfate | 10.0 | - | - | - | - | - | - |
| Hydroxyapatite | - | 20.0 | 0.1 | - | - | - | - |
| Tricalcium phosphate | - | - | - | 20.0 | 0.1 | - | - |
| Calcium monohydrogen phosphate | - | - | - | - | - | 20.0 | 0.1 |
| Palatinit | 40.0 | 40.0 | 40.0 | 40.0 | 40.0 | 40.0 | 40.0 |
| Maltitol | 20.0 | 20.0 | 20.0 | 20.0 | 20.0 | 20.0 | 20.0 |
| Aspartame | 3.0 | 3.0 | 3.0 | 3.0 | 3.0 | 3.0 | 3.0 |
| Dextrin | 2.0 | 2.0 | 2.0 | 2.0 | 2.0 | 2.0 | 2.0 |
| Citric acid | 0.5 | 0.5 | 0.5 | 0.5 | 0.5 | 0.5 | 0.5 |
| Reduced maltose starch syrup | Balance | Balance | Balance | Balance | Balance | Balance | Balance |
| Total | 100.0 | 100.0 | 100.0 | 100.0 | 100.0 | 100.0 | 100.0 |

EP 3 305 275 A1

7. Beverages

[Table 47]

| | Example 145 | Example 146 | Example 147 |
|---|---|---|---|
| Sodium chondroitin sulfate | 0.1 | 0.001 | 1.0 |
| Hydroxyapatite | 0.5 | 0.1 | 0.01 |
| Citric acid | 0.15 | 0.15 | 0.15 |
| Trehalose | 10.0 | 10.0 | 10.0 |
| Lemon flavor | 0.1 | 0.1 | 0.1 |
| Purified water | Balance | Balance | Balance |
| Total | 100.0 | 100.0 | 100.0 |

[Table 48]

| | Example 148 | Example 149 | Example 150 |
|---|---|---|---|
| Sodium chondroitin sulfate | 0.1 | 0.001 | 1.0 |
| Tricalcium phosphate | 0.5 | 0.1 | 0.01 |
| Citric acid | 0.15 | 0.15 | 0.15 |
| Trehalose | 10.0 | 10.0 | 10.0 |
| Lemon flavor | 0.1 | 0.1 | 0.1 |
| Purified water | Balance | Balance | Balance |
| Total | 100.0 | 100.0 | 100.0 |

[Table 49]

| | Example 151 | Example 152 | Example 153 |
|---|---|---|---|
| Sodium chondroitin sulfate | 0.1 | 0.001 | 1.0 |
| Calcium monohydrogen phosphate | 0.5 | 0.1 | 0.01 |
| Citric acid | 0.15 | 0.15 | 0.15 |
| Trehalose | 10.0 | 10.0 | 10.0 |
| Lemon flavor | 0.1 | 0.1 | 0.1 |
| Purified water | Balance | Balance | Balance |
| Total | 100.0 | 100.0 | 100.0 |

[Table 50]

| | Comparative Example 84 | Comparative Example 85 | Comparative Example 86 |
|---|---|---|---|
| Sodium chondroitin sulfate | 1.0 | - | - |
| Hydroxyapatite | - | 0.5 | 0.01 |
| Citric acid | 10.0 | 10.0 | 10.0 |
| Trehalose | 1.0 | 1.0 | 1.0 |

(continued)

|  | Comparative Example 84 | Comparative Example 85 | Comparative Example 86 |
|---|---|---|---|
| Lemon flavor | 10.0 | 10.0 | 10.0 |
| Purified water | Balance | Balance | Balance |
| Total | 100.0 | 100.0 | 100.0 |

[Table 51]

|  | Comparative Example 87 | Comparative Example 88 | Comparative Example 89 | Comparative Example 90 |
|---|---|---|---|---|
| Tricalcium phosphate | 0.5 | 0.01 | - | - |
| Calcium monohydrogen phosphate | - | - | 0.5 | 0.01 |
| Citric acid | 10.0 | 10.0 | 10.0 | 10.0 |
| Trehalose | 1.0 | 1.0 | 1.0 | 1.0 |
| Lemon flavor | 10.0 | 10.0 | 10.0 | 10.0 |
| Purified water | Balance | Balance | Balance | Balance |
| Total | 100.0 | 100.0 | 100.0 | 100.0 |

[Remineralization Test Method]

In order to confirm the remineralizing effect, a previously made artificial incipient caries test sample was used. For the making of the artificial incipient caries test sample, enamel on the labial surface of the crown portion of a bovine front tooth was used. The enamel surface was abraded with #1000, #2400, and #4000 abrasive paper. A window of about $5 \times 3$ mm was made with Nail Enamel (Manufactured by Shiseido Company, Limited) at a site to be tested, on the abraded enamel surface, and immersed in a 0.1 M lactate buffer solution (pH 4.8, 3.0 mmM $CaCl_2$, 1.8 mmM $KH_2PO_4$, 1.0% CMC) at 37°C for 4 days to make artificial incipient caries. For the control for the test, the crown top side half of the window of about $5 \times 3$ mm was further masked with Nail Enamel (Manufactured by Shiseido Company, Limited) to provide a site to be compared (control). A paste dentifrice, a liquid dentifrice, a mouthwash, or a beverage was mixed with purified water to form a suspension solution, and the suspension solution was used as a test solution (test substance). For a chewing gum, a troche, and a candy, after fine grinding, the water-soluble component was extracted from each test substance, and a suspension solution was formed with the water-soluble component and purified water and used as a test solution (test substance).

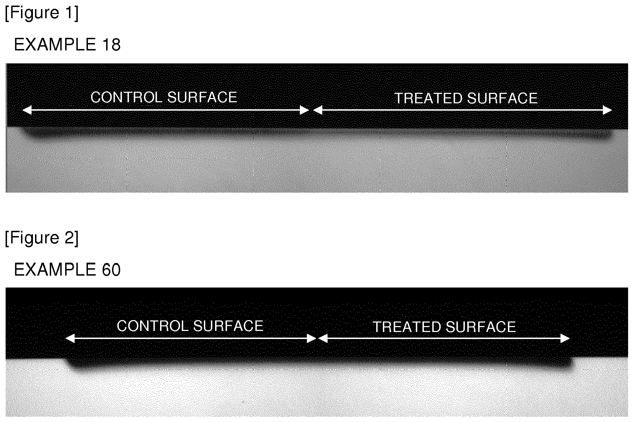

In the remineralization test, the artificial incipient caries specimen made above was immersed in each test solution for 12 days, then the specimen was cut parallel to the tooth axis to a thickness of about 500 $\mu$m by a microcutter, and then this section was abraded under water pouring using #1000, #2400, and #4000 abrasive paper so as to provide a parallel thin section having a thickness of about 100 $\mu$m. After the abrading, contact microradiogram (CMR) photographing was performed (see Figure 1 and Figure 2) in order to check the tooth remineralizing effect. The "CONTROL SURFACE" in each of the Figures is a portion to be compared for to what extent each dentifrice composition in the Examples and Comparative Examples has the remineralizing effect, and the state of the artificial initial caries is maintained. The "CONTROL SURFACE" is a half portion of the artificial initial caries (window of about $5 \times 3$ mm) region. The "TREATED SURFACE" in each of the Figures is a portion in which each test solution (test substance) in the Examples and Comparative Examples is allowed to act.

The effect of remineralizing the artificial incipient caries site was analyzed using a computer.

In image analysis by the computer, the amount of the remineralized mineral was calculated based on the formula of Angmer et al. (B. Angmer, D. Carlstrom and J. E. Glas: Studies on Ultrastructure of Dental Enemel IV: The Mineralization of normal Human Enamel, J. Ultrastructure. Res. 8, 12-23, 1963), and the amounts of the mineral lost $\Delta Z$ (% volume mineral$\cdot\mu$m) at the control surface and treated surface of each section were calculated according to the method of Damato et al. (F. A. Damato, R. Stang and K. W. Stephen: Effect of Fluoride Concentration on Reminerelization of Carious Enamel: an in vitro pH-Cycling Study, Caries Res, 24, 174-180, 1990). The remineralization rate was calculated by the following formula:

[Equation 1]

$$\text{remineralization rate} = \frac{\Delta Z \text{ at control surface} - \Delta Z \text{ at treated surface}}{\Delta Z \text{ at control surface}} \times 100 \ (\%)$$

Table 52 to Table 65 show the results of confirming the remineralizing effect of the dentifrice compositions by such a computer image analysis method.

1. Paste Dentifrices

[Table 52]

| Examples | | | | | | |
|---|---|---|---|---|---|---|
| Example | Example 1 | Example 2 | Example 3 | Example 4 | Example 5 | |
| Remineralization rate (%) | 25.0 | 41.9 | 26.2 | 36.5 | 38.5 | |
| Example | Example 6 | Example 7 | Example 8 | Example 9 | Example 10 | |
| Remineralization rate (%) | 40.2 | 26.9 | 26.8 | 39.1 | 32.2 | |
| Example | Example 11 | Example 12 | Example 13 | Example 14 | Example 15 | Example 16 |
| Remineralization rate (%) | 21.5 | 33.8 | 35.8 | 27.9 | 24.5 | 22.7 |
| Example | Example 17 | Example 18 | Example 19 | Example 20 | Example 21 | |
| Remineralization rate (%) | 37.3 | 30.4 | 24.0 | 31.3 | 25.4 | |
| Example | Example 22 | Example 23 | Example 24 | Example 25 | Example 26 | |
| Remineralization rate (%) | 20.4 | 30.1 | 13.1 | 30.0 | 14.3 | |
| Example | Example 27 | Example 28 | Example 29 | Example 30 | Example 31 | |
| Remineralization rate (%) | 11.0 | 27.0 | 11.5 | 22.4 | 21.8 | |
| Example | Example 32 | Example 33 | Example 34 | Example 35 | Example 36 | |
| Remineralization rate (%) | 26.6 | 13.4 | 13.4 | 26.0 | 15.7 | |
| Example | Example 37 | Example 38 | Example 39 | Example 40 | Example 41 | Example 42 |
| Remineralization rate (%) | 9.3 | 20.8 | 20.2 | 14.3 | 11.3 | 9.8 |
| Example | Example 43 | Example 44 | Example 45 | Example 46 | Example 47 | |
| Remineralization rate (%) | 24.1 | 13.8 | 10.9 | 18.3 | 11.8 | |
| Example | Example 48 | Example 49 | Example 50 | Example 51 | Example 52 | |
| Remineralization rate (%) | 9.3 | 16.8 | 4.9 | 20.9 | 5.4 | |

(continued)

| Example | Example 53 | Example 54 | Example 55 | Example 56 | Example 57 | |
|---|---|---|---|---|---|---|
| Remineralization rate (%) | 11.6 | 27.7 | 12.2 | 24.0 | 25.0 | |
| Example | Example 58 | Example 59 | Example 60 | Example 61 | Example 62 | |
| Remineralization rate (%) | 27.6 | 14.8 | 15.3 | 27.3 | 17.8 | |
| Example | Example 63 | Example 64 | Example 65 | Example 66 | Example 67 | Example 68 |
| Remineralization rate (%) | 10.9 | 23.3 | 24.3 | 16.4 | 12.4 | 11.5 |
| Example | Example 69 | Example 70 | Example 71 | Example 72 | Example 73 | |
| Remineralization rate (%) | 26.7 | 17.2 | 13.9 | 22.8 | 15.9 | |
| Example | Example 74 | Example 75 | Example 76 | Example 77 | Example 78 | |
| Remineralization rate (%) | 14.4 | 23.7 | 10.3 | 26.4 | 10.9 | |

[Table 53]

Comparative Examples

| Example | Comparative Example 1 | Comparative Example 2 | Comparative Example 3 | Comparative Example 4 | Comparative Example 5 | Comparative Example 6 |
|---|---|---|---|---|---|---|
| Remineralization rate (%) | 1.4 | 1.3 | 0.7 | 0.6 | 1.0 | 0.9 |
| Example | Comparative Example 7 | Comparative Example 8 | Comparative Example 9 | Comparative Example 10 | Comparative Example 11 | |
| Remineralization rate (%) | 2.5 | 4.5 | 8.3 | 9.6 | 10.7 | |
| Example | Comparative Example 12 | Comparative Example 13 | Comparative Example 14 | Comparative Example 15 | | |
| Remineralization rate (%) | 11.4 | 12.8 | 13.4 | 14.1 | | |
| Example | Comparative Example 16 | Comparative Example 17 | Comparative Example 18 | Comparative Example 19 | Comparative Example 20 | |
| Remineralization rate (%) | 1.5 | 2.7 | 3.5 | 4.9 | 5.8 | |
| Example | Comparative Example 21 | Comparative Example 22 | Comparative Example 23 | Comparative Example 24 | | |
| Remineralization rate (%) | 6.8 | 7.2 | 7.4 | 7.8 | | |
| Example | Comparative Example 25 | Comparative Example 26 | Comparative Example 27 | Comparative Example 28 | Comparative Example 29 | |
| Remineralization rate (%) | 1.2 | 1.5 | 1.6 | 1.6 | 1.8 | |
| Example | Comparative Example 30 | Comparative Example 31 | Comparative Example 32 | Comparative Example 33 | | |
| Remineralization rate (%) | 2.2 | 2.5 | 2.6 | 2.7 | | |

2. Liquid Dentifrices

[Table 54]

| Examples | | | | | | |
|---|---|---|---|---|---|---|
| Example | Example 79 | Example 80 | Example 81 | Example 82 | Example 83 | Example 84 |
| Remineralization rate (%) | 22.2 | 25.1 | 32.7 | 22.6 | 30.9 | 19.5 |
| Example | Example 85 | Example 86 | Example 87 | Example 88 | Example 89 | Example 90 |
| Remineralization rate (%) | 10.0 | 10.9 | 19.6 | 9.6 | 17.6 | 9.9 |
| Example | Example 91 | Example 92 | Example 93 | Example 94 | Example 95 | Example 96 |
| Remineralization rate (%) | 11.2 | 13.1 | 23.0 | 13.7 | 24.1 | 15.8 |

[Table 55]

| Comparative Examples | | | | | | |
|---|---|---|---|---|---|---|
| Example | Comparative Example 34 | Comparative Example 35 | Comparative Example 36 | | | |
| Remineralization rate (%) | 1.3 | 0.7 | 0.2 | | | |
| Example | Comparative Example 37 | Comparative Example 38 | Comparative Example 39 | Comparative Example 40 | Comparative Example 41 | Comparative Example 42 |
| Remineralization rate (%) | 11.6 | 10.2 | 9.1 | 8.1 | 5.0 | 2.1 |
| Example | Comparative Example 43 | Comparative Example 44 | Comparative Example 45 | Comparative Example 46 | Comparative Example 47 | Comparative Example 48 |
| Remineralization rate (%) | 6.6 | 5.8 | 4.6 | 3.3 | 2.4 | 1.3 |
| Example | Comparative Example 49 | Comparative Example 50 | Comparative Example 51 | Comparative Example 52 | Comparative Example 53 | Comparative Example 54 |
| Remineralization rate (%) | 2.1 | 1.7 | 1.5 | 1.4 | 1.3 | 1.2 |

3. Mouthwashes

[Table 56]

| Examples | | | | |
|---|---|---|---|---|
| Example | Example 97 | Example 98 | Example 99 | Example 100 |
| Remineralization rate (%) | 19.0 | 28.3 | 18.5 | 18.0 |
| Example | Example 101 | Example 102 | Example 103 | Example 104 |
| Remineralization rate (%) | 6.8 | 13.2 | 8.6 | 8.2 |
| Example | Example 105 | Example 106 | Example 107 | Example 108 |
| Remineralization rate (%) | 10.4 | 20.9 | 14.4 | 14.9 |

[Table 57]

| Comparative Examples | | | | | | |
|---|---|---|---|---|---|---|
| Example | Comparative Example 55 | Comparative Example 56 | | | | |
| Remineralization rate (%) | 1.2 | 1.1 | | | | |
| Example | Comparative Example 57 | Comparative Example 58 | Comparative Example 59 | Comparative Example 60 | Comparative Example 61 | Comparative Example 62 |
| Remineralization rate (%) | 2.2 | 7.5 | 1.5 | 3.1 | 1.5 | 1.3 |

4. Chewing gums

[Table 58]

| Examples | | | |
|---|---|---|---|
| Example | Example 109 | Example 110 | Example 111 |
| Remineralization rate (%) | 23.9 | 32.9 | 26.5 |
| Example | Example 112 | Example 113 | Example 114 |
| Remineralization rate (%) | 10.1 | 15.4 | 13.2 |
| Example | Example 115 | Example 116 | Example 117 |
| Remineralization rate (%) | 15.0 | 21.2 | 15.2 |

[Table 59]

Comparative Examples

| Example | Comparative Example 63 | Comparative Example 64 | Comparative Example 65 | Comparative Example 66 | Comparative Example 67 | Comparative Example 68 | Comparative Example 69 |
|---|---|---|---|---|---|---|---|
| Remineralization rate (%) | 0.9 | 8.4 | 11.1 | 3.4 | 6.4 | 1.5 | 2.0 |

5. Troches

[Table 60]

| Examples | | | | | |
|---|---|---|---|---|---|
| Example | Example 118 | Example 119 | Example 120 | Example 121 | Example 122 |
| Remineralization rate (%) | 36.7 | 27.4 | 20.4 | 28.1 | 20.3 |
| Example | Example 123 | Example 124 | Example 125 | Example 126 | Example 127 |
| Remineralization rate (%) | 18.2 | 13.8 | 8.1 | 17.4 | 10.2 |
| Example | Example 128 | Example 129 | Example 130 | Example 131 | Example 132 |
| Remineralization rate (%) | 22.2 | 15.5 | 10.6 | 22.7 | 15.8 |

[Table 61]

Comparative Examples

| Example | Comparative Example 70 | Comparative Example 71 | Comparative Example 72 | Comparative Example 73 | Comparative Example 74 | Comparative Example 75 | Comparative Example 76 |
|---|---|---|---|---|---|---|---|
| Remineralization rate (%) | 1.0 | 13.2 | 3.9 | 7.5 | 1.7 | 2.5 | 1.4 |

6. Candies

[Table 62]

| Examples | | | | |
|---|---|---|---|---|
| Example | Example 133 | Example 134 | Example 135 | Example 136 |
| Remineralization rate (%) | 29.7 | 34.5 | 34.7 | 22.0 |
| Example | Example 137 | Example 138 | Example 139 | Example 140 |
| Remineralization rate (%) | 15.6 | 21.5 | 19.0 | 8.8 |
| Example | Example 141 | Example 142 | Example 143 | Example 144 |
| Remineralization rate (%) | 17.0 | 23.6 | 24.0 | 11.9 |

[Table 63]

| Comparative Examples | | | | | | | |
|---|---|---|---|---|---|---|---|
| Example | Comparative Example 77 | Comparative Example 78 | Comparative Example 79 | Comparative Example 80 | Comparative Example 81 | Comparative Example 82 | Comparative Example 83 |
| Remineralization rate (%) | 0.2 | 12.8 | 8.3 | 7.0 | 3.4 | 2.5 | 1.4 |

7. Beverages

[Table 64]

| Examples | | | |
|---|---|---|---|
| Example | Example 145 | Example 146 | Example 147 |
| Remineralization rate (%) | 34.3 | 23.6 | 22.2 |
| Example | Example 148 | Example 149 | Example 150 |
| Remineralization rate (%) | 18.4 | 10.2 | 10.9 |
| Example | Example 151 | Example 152 | Example 153 |
| Remineralization rate (%) | 23.9 | 14.5 | 15.8 |

[Table 65]

| Comparative Examples | | | | | | | |
|---|---|---|---|---|---|---|---|
| Example | Comparative Example 84 | Comparative Example 85 | Comparative Example 86 | Comparative Example 87 | Comparative Example 88 | Comparative Example 89 | Comparative Example 90 |
| Remineralization rate (%) | 1.4 | 9.2 | 5.5 | 4.3 | 2.1 | 1.4 | 1.2 |

As shown in the Comparative Examples in which sodium chondroitin sulfate in the paste dentifrices, the liquid dentifrices, the mouthwashes, the chewing gums, the troches, the candies, and the beverages is blended alone, sodium chondroitin sulfate alone has little tooth remineralizing effect.

In contrast to this, when sodium chondroitin sulfate is used together with hydroxyapatite, tricalcium phosphate, or calcium monohydrogen phosphate, the synergistic improving effect of the tooth remineralizing effect is seen compared with when hydroxyapatite, tricalcium phosphate, or calcium monohydrogen phosphate is used alone. The total remineralization rate described below is a value obtained by adding remineralization rates in Comparative Examples. Specifically, from the comparisons of, for example,

Example 1 (remineralization rate: 25.0%) with Comparative Example 1 and Comparative Example 15 (total remineralization rate: 15.5%),

Example 5 (remineralization rate: 38.5%) with Comparative Example 3 and Comparative Example 14 (total remineralization rate: 14.1%),

Example 15 (remineralization rate: 24.5%) with Comparative Example 5 and Comparative Example 11 (total remineralization rate: 11.7%),

Example 46 (remineralization rate: 18.3%) with Comparative Example 2 and Comparative Example 18 (total remineralization rate: 4.8%),

Example 66 (remineralization rate: 16.4%) with Comparative Example 4 and Comparative Example 29 (total remineralization rate: 2.4%),

Example 80 (remineralization rate: 25.1%) with Comparative Example 36 and Comparative Example 38 (total remineralization rate: 10.4%),

Example 85 (remineralization rate: 10.0%) with Comparative Example 34 and Comparative Example 43 (total remineralization rate: 7.9%),

Example 96 (remineralization rate: 15.8%) with Comparative Example 35 and Comparative Example 54 (total remineralization rate: 1.9%),

Example 97 (remineralization rate: 19.0%) with Comparative Example 55 and Comparative Example 58 (total remineralization rate: 8.7%),

Example 108 (remineralization rate: 14.9%) with Comparative Example 56 and Comparative Example 61 (total remineralization rate: 2.6%),

Example 109 (remineralization rate: 23.9%) with Comparative Example 63 and Comparative Example 64 (total remineralization rate: 9.3%),

Example 132 (remineralization rate: 15.8%) with Comparative Example 70 and Comparative Example 76 (total remineralization rate: 2.4%),

Example 140 (remineralization rate: 8.8%) with Comparative Example 77 and Comparative Example 81 (total remineralization rate: 3.6%), and

Example 147 (remineralization rate: 22.2%) with Comparative Example 84 and Comparative Example 86 (total remineralization rate: 6.9%),

the synergistic effect is clear.

**Industrial Applicability**

The oral composition, food, or beverage of the present invention are very excellent in the remineralization of teeth and have high industrial usefulness.

**Claims**

**1.** An oral composition, a food, or a beverage comprising sodium chondroitin sulfate and calcium phosphate.

**2.** The oral composition, the food, or the beverage according to claim 1, wherein the calcium phosphate is at least one calcium phosphate selected from the group consisting of hydroxyapatite, tricalcium phosphate, and calcium monohydrogen phosphate.

**3.** The oral composition, the food, or the beverage according to claim 1 or 2, wherein a content of the calcium phosphate is 0.0001 to 40% by mass.

**4.** The oral composition, the food, or the beverage according to any one of claims 1 to 3, wherein a content of the sodium chondroitin sulfate is 0.0001 to 15% by mass.

**5.** The oral composition according to any one of claims 1 to 4, wherein the composition is a paste dentifrice, a powder dentifrice, a liquid dentifrice, a mouthwash, an oral cleaning agent, or a troche.

**6.** The food according to any one of claims 1 to 4, wherein the food is a chewing gum, a candy, a chocolate, a yogurt, or a jelly.

**7.** The beverage according to any one of claims 1 to 4, wherein the beverage is a carbonated beverage, a lactic acid bacteria beverage, a fruit juice beverage, or a juice.

**8.** The oral composition, a food, or a beverage according to any one of claims 1 to 7 for use in the remineralization of teeth.

[Figure 1]

EXAMPLE 18

[Figure 2]

EXAMPLE 60

[Figure 3]

BLACK REGION OUTSIDE
ENAMEL SURFACE LAYER
IS SPACE (BACKGROUND)

(EXAMPLE OF CMR
PHOTOGRAPHING)

CONTROL SURFACE             TREATED SURFACE

GRAY BORDERLINE IS
SURFACE OF "ENAMEL
SURFACE LAYER"

GRAY REGION IS
"HEALTHY ENAMEL"

"INCIPIENT CARIES REGION" IN
WHICH ENAMEL SURFACE LAYER IS
ARTIFICIALLY DEMINERALIZED

## INTERNATIONAL SEARCH REPORT

International application No.

PCT/JP2016/002470

A. CLASSIFICATION OF SUBJECT MATTER
*A61K8/73*(2006.01)i, *A23G1/00*(2006.01)i, *A23G1/30*(2006.01)i, *A23G3/34*
(2006.01)i, *A23G4/00*(2006.01)i, *A23L2/52*(2006.01)i, *A23L33/10*(2016.01)i,
*A23L33/16*(2016.01)i, *A61K8/24*(2006.01)i, *A61Q11/00*(2006.01)i
According to International Patent Classification (IPC) or to both national classification and IPC

B. FIELDS SEARCHED

Minimum documentation searched (classification system followed by classification symbols)
A61K8/73, A23G1/00, A23G1/30, A23G3/34, A23G4/00, A23L2/52, A23L33/10,
A23L33/16, A61K8/24, A61Q11/00

Documentation searched other than minimum documentation to the extent that such documents are included in the fields searched
Jitsuyo Shinan Koho        1922–1996   Jitsuyo Shinan Toroku Koho   1996–2016
Kokai Jitsuyo Shinan Koho   1971–2016   Toroku Jitsuyo Shinan Koho   1994–2016

Electronic data base consulted during the international search (name of data base and, where practicable, search terms used)
JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| X | JP 2002-325557 A  (Ezaki Glico Co., Ltd.), 12 November 2002 (12.11.2002), claims; paragraphs [0058], [0092], [0402]; examples 12, 39, 41 to 43, 48, 49, 55, 57 to 59, 64, 65 & US 2004/0105823 A1 claims; paragraphs [0096], [0128], [0445]; examples 12, 39, 41 to 43, 48, 49, 55, 57 to 59, 64, 65 & WO 2002/067871 A2     & EP 1363580 A2 & DE 60219438 T2        & CN 1501774 A & KR 10-0911382 B1 | 1-8 |
| X | JP 7-53388 A  (Lion Corp.), 28 February 1995 (28.02.1995), claims; paragraphs [0011], [0013]; example 12 (Family: none) | 1-7 |

| ☒ | Further documents are listed in the continuation of Box C. | ☐ | See patent family annex. |
|---|---|---|---|

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search 06 June 2016 (06.06.16) | Date of mailing of the international search report 05 July 2016 (05.07.16) |
|---|---|
| Name and mailing address of the ISA/ Japan Patent Office 3-4-3,Kasumigaseki,Chiyoda-ku, Tokyo 100-8915,Japan | Authorized officer Telephone No. |

Form PCT/ISA/210 (second sheet) (January 2015)

**INTERNATIONAL SEARCH REPORT**

International application No.

PCT/JP2016/002470

C (Continuation). DOCUMENTS CONSIDERED TO BE RELEVANT

| Category* | Citation of document, with indication, where appropriate, of the relevant passages | Relevant to claim No. |
|---|---|---|
| X | JP 63-253018 A (Sunstar Inc.), 20 October 1988 (20.10.1988), claims; page 3, lower left column, lines 16 to 18; examples 3, 4 (Family: none) | 1,2,4-7 |
| X | JP 2008-273919 A (Lilac Co., Ltd.), 13 November 2008 (13.11.2008), paragraph [0013]; preparation example 7 (Family: none) | 1-7 |
| X | JP 7-308169 A (Kabushiki Kaisha Haputo International), 28 November 1995 (28.11.1995), claims; paragraph [0017] (Family: none) | 1-7 |
| X | JP 62-240605 A (Nobuo SHIODA), 21 October 1987 (21.10.1987), examples 1, 6; page 4, lower right column, lines 8 to 12 (Family: none) | 1-7 |
| X | Ichiro TSUJI, Masataka YOSHIKAWA, "Rinsan 4 Calcium - Chondroitin Ryusan A Gozai no Konkan Jutenzai to shiteno Bussei to Soshiki Shigekisei", Nihon Shika Hozongaku Zasshi, 1993.06, vol.36, no.3, pages 845 to 867 | 1-4 |
| A | WO 2006/038315 A1 (Sangi Co., Ltd.), 13 April 2006 (13.04.2006), & US 2007/0166362 A1 & EP 1797900 A1 & CN 1838969 A & KR 10-2007-0060950 A | 1-8 |
| A | JP 1-213222 A (Warner-Lambert Co.), 28 August 1989 (28.08.1989), & US 4855128 A & EP 324720 A1 & AU 2664088 A | 1-8 |
| A | CN 102973980 A (Fuzhou University), 20 March 2013 (20.03.2013), (Family: none) | 1-8 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 9202717 A
- JP 10059814 A
- JP 2000128752 A
- JP 2001122748 A
- JP 2005314266 A
- JP 2014073989 A
- JP 2006213668 A
- JP 2007099632 A
- JP 7223930 A
- JP 63253018 A
- JP 2006117563 A
- JP 2009046449 A
- JP 2009196987 A

**Non-patent literature cited in the description**

- **B. ANGMER ; D. CARLSTROM ; J. E. GLAS.** Studies on Ultrastructure of Dental Enemel IV. *The Mineralization of normal Human Enamel, J. Ultrastructure. Res.,* 1963, vol. 8, 12-23
- **F. A. DAMATO ; R. STANG ; K. W. STEPHEN.** Effect of Fluoride Concentration on Reminerelization of Carious Enamel. *an in vitro pH-Cycling Study, Caries Res,* 1990, vol. 24, 174-180